# EUROPEAN PATENT SPECIFICATION

(11) **EP 3 393 243 B1**
(45) Date of publication and mention of the grant of the patent: **02.03.2022**
(21) Application number: 16822830.2
(22) Date of filing: 08.12.2016
(51) Int. Cl.: A01N 37/18, A01N 59/16, A61K 31/16, A61K 33/24, A61K 33/40

(54) **COMPOSITIONS COMPRISING AN AMIDE**
ZUSAMMENSETZUNGEN MIT EINEM AMID
COMPOSITIONS COMPRENANT UN AMIDE

(30) Priority: 22.12.2015 US 201562271029 P
(43) Date of publication of application: 31.10.2018
(73) Proprietor: The Procter & Gamble Company, Cincinnati, OH 45202 (US)
(72) Inventor: SHERRY, Alan, Edward, Cincinnati, Ohio 45202 (US); BOHLEN, Molly, Pelon, Cincinnati, Ohio 45202 (US); NJOROGE, Samuel, Kimani, Cincinnati, Ohio 45202 (US); GLENN, LaShanda, Marie, Cincinnati, Ohio 45202 (US); DHONAU, Janice, Lynn, Cincinnati, Ohio 45202 (US); MORRISSEY, Christopher, Todd, Cincinnati, Ohio 45202 (US); FITZGERALD, Jamesina, Anne, Cincinnati, Ohio 45202 (US); VINSON, Phillip, Kyle, Cincinnati, Ohio 45202 (US); STEFFEY, Melinda, Phyllis, Cincinnati, Ohio 45202 (US); ORTIZ, Rafael, Cincinnati, Ohio 45202 (US); HALE, Benjamin, Patrick, Cincinnati, Ohio 45202 (US)
(74) Representative: P&G Patent Belgium UK
(86) International application number: PCT/US2016/065597
(87) International publication number: WO 2017/112425

(56) References cited:
- US-A1- 2003 099 717
- US-A1- 2012 295 831
- BISTLINE R G ET AL: "FATTY ACID AMIDES AND ANILIDES, SYNTHESES AND ANTIMICROBIAL PROPERTIES", 1 February 1980 (1980-02-01), JOURNAL OF THE AMERICAN OIL CHEMISTS' SOCIETY (JAOCS), SPRINGER, DE, PAGE(S) 98 - 103, XP009012473, ISSN: 0003-021X Results and Discussion; page 103, last paragraph; table 1

## Description

### FIELD OF THE INVENTION

The present disclosure relates to compositions containing an amide, products incorporating the compositions, and methods of using the compositions and products.

### BACKGROUND OF THE INVENTION

The past decade has ushered an era of unprecedented advances in aqueous antimicrobial chemistry. Much effort has been directed at supplementing the antimicrobial activity of compositions comprising a combination of surfactant, organic acid, and either a North American registered active or a notified active recognized by the European Biocidal Products Regulation. At the same time, increased environmental awareness has created new demand for compositions based on renewable actives. The net result has been a proliferation of commercially available antimicrobial products based on organic acids, essential oils, silver compounds, and hydrogen peroxide that promise increasing cidal speed and broad spectrum activity. Given the limited number of government-accepted antimicrobial actives, advances have required the identification of safe, non-active raw materials, which, when combined with known antimicrobials, result in potentiation of antimicrobial activity.

Saturated and unsaturated, linear, branched, and cyclic amides are known in the art, and processes for making these amides are known. Cleaning compositions comprising an anionic surfactant salt, a saturated C₈ to C₁₀ alkyl amide solvent, a cosolvent, and water, where the compositions are substantially free of d-limonene and have a pH of between 6.5 and 10, are known. Aqueous hard surface cleaners comprising water, a monounsaturated C₈-C₁₄ fatty N,N-dialkylamide, and at least one anionic, cationic, nonionic, or amphoteric surfactant.

Compositions comprising a mixture of 2-(thiocyanomethylthio)benzothiazole and methylene-bis(thiocyanate) with dimethylamide of a carboxylic acid, which are synergistically effective compared to the respective components alone in controlling the growth of microorganisms, are known. Antimicrobial compositions comprising ionic silver, hydrogen peroxide, or a mixture thereof are also known. For example, aqueous disinfectants formulated by electrolytically generating silver ions in water in combination with a citric acid are known. Sterilizing and virus-removing actives containing hydrogen peroxide, benzyl alcohol and a silver component are known. Acidic aqueous disinfecting solutions comprising hydrogen peroxide, an anionic surfactant, and benzyl alcohol are known. Also, a disinfectant composition including a peroxide, a peracid, an anionic surfactant, a nonionic polymer, one or both of a linear fatty alcohol and an alkyl pyrrolidone is known.

US 2003/0099717 A1 discloses a wide spectrum disinfecting and antiseptic composition for use in the fields of human medicine, veterinary science and industry, characterized because it includes:
Hydrogen peroxide, lactic acid and halogen salts (Br, I) and/or salts of heavy metals (for example, silver halides) with surfactant agents, either cationic, like chlorhexidine and/or quaternary ammonium salts, like didecyl-methyl-polyoxy-ethyl-ammonium propionate, chlorides of ammonium or compounds of ammonium propylamide or anionic, like lauryl sulphate, dodecyl sulphate or alkyl succinic salts, with suitable excipients, some of which may be ethyl or isopropyl alcohol, chlorhexidine, non-chlorinated quaternary ammonium salts, like didecyl-methyl-polyoxy-ethyl-ammonium propionate, combined or not with iodine, and/or its salts, together with excipients, some of which may be ethyl or isopropyl alcohol.

There remains a need for a composition that provides improved cleaning as well as antimicrobial benefits, while comprising an increased content of raw materials derived from renewable sources. It is not known to include one or more amides in a composition to potentiate antimicrobial activity. It has now surprisingly been found that amides (which may be derived from renewable sources) may be incorporated into phase stable aqueous solutions to provide cleaning and potentiation of antimicrobial activity.

### SUMMARY OF THE INVENTION

The present disclosure attempts to solve one or more of the needs above by providing a composition comprising a surfactant; an acidifying agent; an amide of formula I:

R¹-CO-NR²R³ (I)

wherein R¹ is selected from the group consisting of linear or branched, substituted or unsubstituted C₆-C₁₂ hydrocarbyl groups, each of R² and R³ is independently selected from H, OH, a halogen, or C₁-C₆ linear or branched, substituted or unsubstituted hydrocarbyl groups; and water; wherein said composition has a pH from 1.0 to 6.0 wherein said composition is an antimicrobial composition comprising from 0.01% to 30% of an antimicrobial active, wherein the antimicrobial active is an active oxygen source, wherein the active oxygen source is hydrogen peroxide, and the active oxygen source is present at a level of from 0.05% to 8% by weight of the composition.

### DETAILED DESCRIPTION OF THE INVENTION

Features and benefits of the disclosed invention will become apparent from the following description, which includes examples intended to give a broad representation of the invention.

As used herein, the articles including "the," "a" and "an" when used in a claim or in the specification, are understood to mean one or more of what is claimed or described.

As used herein, the terms "include," "includes" and "including" are meant to be non-limiting.

As used herein, the terms "active" and "agent" are used interchangeably.

As used herein, the term "renewable" (as in "renewable feedstock") refers to materials (e.g., surfactant, solvent, acidifying agent) that are derived from a renewable feedstock and contain renewable carbon. The term "renewable" is used interchangeably with the terms "biobased" and "natural." A renewable feedstock is a feedstock that is derived from a renewable resource, e.g., plants, and non-geologically derived. A material may be partially renewable (less than 100% renewable carbon content), 100% renewable (100% renewable carbon content), or somewhere in between (e.g., 50% renewable carbon content). A renewable material, for example a renewable ethanol, may be blended with a non-renewable material, for example, conventional ethanol, to yield a partially renewable material, e.g., partially renewable ethanol.

The terms "microorganism" or "microbe" as used herein are intended to include cellular organisms, both unicellular and multicellular that are less than 5 mm in length, and include but are not limited to bacteria, fungi, prions, enveloped and non-enveloped viruses, archaea, protists, protozoa or oocysts formed by protozoa, green algae, plankton, planarian, amoebas and yeasts, or spores formed by any of these. The terms "microorganism" or "microbe" include the single or planktonic microbes that may contaminate surfaces, as well as communities of microbes that grow as biofilms on surfaces.

The term "antimicrobial" as used herein refers to a compound that exhibits microbicide or microbiostatic properties that enables the compound to kill, destroy, inactivate, or neutralize a microorganism; or to mitigate, prevent, or reduce the growth, ability to survive, or propagation of a microorganism. In the context of antimicrobial, the term "treat" means to kill, destroy, inactivate, or neutralize a microorganism; or to prevent or reduce the growth, ability to survive, or propagation of a microorganism.

The term "substantially free of' or "substantially free from" as used herein refers to either the complete absence of an ingredient or a minimal amount thereof merely as impurity or unintended byproduct of another ingredient. A composition that is "substantially free" of/from a component means that the composition comprises less than 0.01%, or less than 0.001%, or even 0%, by weight of the composition, of the component.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The citation of any patent or other document is not an admission that the cited patent or other document is prior art with respect to the present invention.

In this description, all concentrations and ratios are on a weight basis of the composition unless otherwise specified.

Unless otherwise noted, all component or composition levels are in reference to the active portion of that component or composition, and are exclusive of impurities, for example, residual solvents or by-products, which may be present in commercially available sources of such components or compositions.

All measurements are performed at 25°C unless otherwise specified.

### Composition

The compositions of the present disclosure may be formulated as concentrates or ready-to-use compositions. The compositions of the present disclosure may deliver cleaning benefits on hard and soft surfaces. The compositions of the present disclosure may be antimicrobial compositions and deliver improved antimicrobial activity on hard and soft surfaces. The compositions of the present disclosure may deliver cleaning benefits as well as antimicrobial benefits on hard and soft surfaces. The compositions disclosed herein may also be made in whole or in part using raw materials derived from renewable feedstocks, such as plant oils.

Concentrates may be diluted with water in order to provide an in-use solution having a desired level of detersive properties or other properties, including antimicrobial properties. The antimicrobial properties desired may depend on the challenge posed by the target microorganism; for example, enveloped viruses are more susceptible to inactivation than non-enveloped viruses, and spore-forming organisms are very resistant to chemical inactivation. Each organism type presents a different challenge and may call for a different level of dilution (or none) in order to achieve the desired antimicrobial activity.

The water used to dilute the concentrate (water of dilution) can be available at the locale or site of dilution. The water of dilution may contain varying levels of hardness depending upon the locale. Service waters available from various municipalities have varying levels of hardness. It is desirable to provide a concentrate that can handle the hardness levels found in the service water of various municipalities. The water of dilution may have a hardness ranging from zero to at least 400 ppm hardness (as CaCO₃).

Concentrated solutions may provide for improved economics to the manufacturer and to the user as they may comprise less water and may use less packaging material on a per-use basis, as compared to a ready-to-use composition. A concentrated composition may be diluted with water at a weight ratio of composition to water ranging from 1:1.5 to 1:1000, or from 1:4 to 1:250. The terms "in-use composition" or "in-use diluted composition" refer to concentrated compositions that have been diluted with water prior to use.

Alternatively, the compositions may be ready-to-use antimicrobial sprays, gels, creams, powders, soluble unit dose articles, pastes, or antimicrobial wet wipes. The compositions may also be comprised into foams, such as melamine-formaldehyde foams. For toilet hygiene, the compositions can be in the form of dissolvable rim-blocs which partially dissolve after the toilet is flushed. Ready to use compositions may provide more convenience to the user.

The compositions disclosed herein generally provide short-contact-time antimicrobial benefits, e.g., from 10 seconds to 3 minutes or from 15 seconds to 2 minutes, or from 30 seconds to 1 minute, though longer contact times, e.g., from 3 minutes to 15 minutes, may also be achieved

### Surfactant

The compositions of the present disclosure may comprise one or more surfactants. The surfactant may comprise from 6 to 12 carbon atoms, or from 6 to 11 carbon atoms, or from 6 to 10 carbon atoms, or from 8 to 10 carbon atoms. The surfactant may be branched or linear, saturated or unsaturated. The surfactant may be branched and comprise from 6 to 12 carbon atoms, or from 6 to 11 carbon atoms, or from 6 to 10 carbon atoms, or from 8 to 10 carbon atoms in the primary carbon chain, where "primary carbon chain" denotes the longest carbon-based chain that is uninterrupted by a heteroatom, such as O, S, N and P. For example, *n*-octyl sulfate has 8 carbon atoms in the primary carbon chain, 2-propyl-1-heptyl sulfate has 7 carbon atoms in the primary carbon chain, and dodecyl methyl ester sulfonate (C₁₀H₂₁-CH(SO₃⁻)-C(O)O-CH₃) has 11 carbon atoms in the primary carbon chain. In the context of branched surfactants, Cₙ (such as C₁ or C₈) refers to the number of carbon atoms in the primary carbon chain (for example, a 2-ethyl-1-hexyl primary carbon chain is C₆). In the context of linear (or unbranched) surfactants, Cₙ (such as C₁ or C₈) refers to the total number of carbon atoms in the surfactant.

The surfactants may be substantially free of trace transition metal impurities (particularly for antimicrobial compositions comprising hydrogen peroxide). The surfactants may be substantially free of trace levels of chloride, bromide, and iodide (particularly for antimicrobial compositions comprising ionic silver).

The compositions may comprise from 0.01% to 60%, or from 0.01% to 40%, or from 0.03% to 35%, or from 0.05% to 30% of surfactant.

The concentrated compositions disclosed herein are generally intended to be diluted prior to use. A concentrated composition may comprise from 0.5% to 1%, or from 1% to 2%, or from 2% to 3%, or from 3% to 5%, or from 5% to 10%, or from 10% to 20%, or from 20% to 40%, of surfactant. A ready-to-use antimicrobial composition may comprise from 0.01% to 0.05%, or from 0.05% to 0.10%, or from 0.10% to 0.15%, or from 0.15% to 0.25%, or from 0.25% 0.50% of surfactant. Ready-to-use antimicrobial compositions may comprise greater concentrations of surfactant, e.g., greater than 0.50% (for example, for treatment of surfaces contaminated with mycobacteria, spore forming organisms, or biofilms).

Without being bound by theory, it is believed that the short chain-length of the surfactant - from 6 to 12 carbon atoms, or from 6 to 11 carbon atoms, or from 6 to 10 carbon atoms, or from 8 to 10 carbon atoms - is particularly beneficial for antimicrobial applications involving shorter contact times between the microorganism and the composition, for example, from 10 seconds to 3 minutes or from 15 seconds to 2 minutes, or from 30 seconds to 1 minute. The short chain-length of the surfactant is believed to enhance the activity of antimicrobial active(s) in the composition. The short chain-length of the surfactant is also believed to help solubilize the (otherwise substantially water-insoluble) amide. Critical Micelle Concentration (CMC) measurements in the presence and absence of the amide indicate that the surfactants disclosed herein enhance the solubility of the amide by incorporating the amide into the micellar structure(s) of the surfactant. The CMC of the surfactant is significantly reduced, and this provides a reservoir of solubilized amide for antimicrobial potentiation activity. It is believed that the chain-length of the surfactant and the chain-length of the amide may be matched, for example, where the difference between the chain-length of the surfactant and the chain-length of the amide is 2 to 3 carbon atoms, to provide a combination of increased solubility of the amide in the composition and increased antimicrobial activity of the composition.

The compositions disclosed herein may comprise one or more C₁₃₋₁₈ surfactants. For example, commercial surfactants are generally made up of a blend of molecules having different alkyl chain lengths (though it is possible to obtain single chain-length cuts), e.g., Polystep^{®} B-25 (from the Stepan Company) is described as sodium decyl sulfate but also contains 25%-30% dodecyl sulfate, by weight of the alkyl sulfate surfactant. Similarly, many commercial lauryl surfactants may include 30% or more surfactant having chain-length(s) greater than C₁₂. When C₁₃₋₁₈ surfactant is present in the composition(s), the weight ratio of C₆₋₁₂ surfactant to C₁₃₋₁₈ surfactant may be greater than 2:1, or greater than 3:1. The average chain-length of the surfactant in the composition(s) may be less than C₁₂, or less than C₁₁. The surfactant in the composition may have an average chain-length of from C₇ to C₈, or from C₈ to C₉, or from C₉ to C₁₀, or from C₁₀ to C₁₁. The composition(s) may comprise surfactant having an average chain-length of C₈.

The solubility of the amide may be further increased by utilizing C₁₃₋₁₈ surfactants. However, increased surfactant chain-length further reduces CMC, which means that a reduced concentration of both surfactant monomer and amide monomer is available for antimicrobial potentiation. Thus, there may be an optimal balance of shorter chain-length surfactant and longer chain-length surfactant, whereby the longer chain-length surfactant helps to solubilize the amide via formation of mixed micelles, and the shorter chain-length surfactant increases the CMC of the surfactant and the concentration of surfactant monomers and amide monomers that drive short-contact-time antimicrobial activity. The optimal balance may vary, depending on whether short-contact-time antimicrobial activity is desired. In other words, increased CMC values may be advantageous for faster activity, while reduced CMC values may be advantageous for longer-contact-time applications.

The antimicrobial compositions disclosed herein may comprise a surfactant comprising from 6 to 12 carbon atoms and an amide of formula I (comprising from 6 to 12 carbon atoms). The CMC of the composition may be from 100 ppm to 2,500 ppm, or from 200 ppm to 2,000 ppm, or from 300 ppm to 1,500 ppm.

The antimicrobial composition(s) disclosed herein may comprise surfactant selected from the group consisting of an anionic surfactant, a cationic surfactant, a nonionic surfactant, an amphoteric surfactant, a zwitterionic surfactant, and mixtures thereof.

Suitable anionic surfactants include the sodium, potassium, ammonium, alkanolammonium magnesium and calcium salts of C₈-C₁₀ glyceryl ether sulfonates, C₈ alkyl sulfonates, C₂-C₈ linear alkyl benzene sulfonate, C₆-C₁₂ alkyl sulfates, C₈-C₁₂ alkyl ether sulfates, C₅₋₁₀ alkyl and alkenyl succinates as mono or dianionic surfactants [e.g., R- CH(COO⁻M⁺)-CH₂-COO⁻ M⁺, R-CH(COO⁻ M⁺)-CH₂-COOH ⁻ and R-CH(COOH)-CH₂-COO⁻ M⁺ wherein R = C₅₋₁₀ linear or branched alkyl or alkenyl group and M = lithium, sodium, potassium, ammonium or alkanolammonium, and mixtures thereof], C₈-C₁₂ methyl ester sulfonates, C₈-C₁₂ fatty acid sulfonates and C₆-C₁₂ carboxylates, and mixtures thereof. The surfactant may comprise an anionic surfactant selected from the group consisting of sodium octyl sulfate, sodium decyl sulfate, sodium octyl glyceryl ether sulfonate (C₈H₁₇-O-CH₂-CH(OH)-CH₂SO₃Na), the sodium salt of 2-propyl-1-heptyl sulfate, the sodium salts of C₉₋₁₁ secondary sulfates, the sodium salts of C₁₂ methyl ester sulfonate and C₁₂ fatty acid sulfonate, and mixtures thereof. The surfactant may comprise an anionic surfactant selected from the group consisting of octyl sulfate, sodium decyl sulfate, and mixtures thereof. The anionic surfactant may be derived from a renewable feedstock, for example, *n*-octyl alcohol or *n*-decyl alcohol derived from plant oils for the making of *n*-octyl sulfate and *n*-decyl sulfate, respectively, and *n*-dodecyl methyl ester derived from plant oils for the making of C₁₂ methyl ester sulfonate and C₁₂ fatty acid sulfonate.

Suitable nonionic surfactants include linear or branched, saturated or unsaturated alcohol alkoxylates, alkyl glycosides, and alkyl ethoxy carboxylic acids comprising from 6 to 12 carbon atoms in the primary chain. The surfactant may comprise a nonionic surfactant selected from the group consisting of C₆₋₁₂ alcohol ethoxylate comprising an average of from 1 mole to 7 moles of ethylene oxide, C₆₋₁₂ alcohol ethoxy propoxylate comprising an average of from 1 mole to 7 moles of ethylene oxide and from 1 mole to 4 moles of propylene oxide, C₈ pyrrolidone, C₈ and C₈₋₁₀ alkyl polyglucoside with a degree of glucoside polymerization of from 1 to 1.6, C₈₋₁₀ alkyl polypentoside (e.g., xyloside and riboside) with a degree of sugar pentoside polymerization of from 1 to 1.6, and C₁₂ ethoxy carboxylic acid comprising an average of from 1 mole to 3 moles of ethylene oxide. The surfactant may comprise a nonionic surfactant selected from the group consisting of octyl alkylpolyglycoside, decyl alkylpolyglycoside, octyl pyrrolidone, and mixtures thereof. As in the case of the anionic surfactant, the nonionic surfactant may be derived from a renewable feedstock. For example, the C₈ and C₈₋₁₀ alkyl polyglycosides (hexosides and pentosides) may be made from entirely renewable feedstocks.

Suitable cationic surfactants include saturated or unsaturated betaines, amine oxides, alkyl morpholinium compounds and alkyl trimethyl ammonium compounds comprising from 6 to 12 carbon atoms. The surfactant may comprise a cationic surfactant selected from the group consisting of *n*-octyl dimethyl amine oxide, *n*-octyl dimethyl betaine, *n*-octyl amidopropyl betaine, and mixtures thereof. The cationic surfactant may be derived from a renewable feedstock.

Suitable zwitterionic surfactants include 2-ethyl-1-hexyl imino dipropionate as well as *n-*dodecyl imino dipropionate (mono- and dianionic salts), C₆₋₁₂ amphoglycinates, and C₆₋₁₂ alkyl sulfobetaines, such as the sodium salt of *n*-octyl, *n*-decyl, or *n*-dodecyl N,N-dimethyl-3-ammonio-1-propanesulfonate.

The antimicrobial composition(s) disclosed herein may comprise surfactant selected from the group consisting of C₈ glyceryl ether sulfonate, C₆-C₁₂ alkyl sulfate, C₈-C₁₂ methyl ester sulfonate, C₈-C₁₂ fatty acid sulfonate, C₆-C₁₂ ether carboxylate, C₈₋₁₀ amine dimethyl oxide, C₈ pyrrolidone, C₈ dimethyl betaine, C₈₋₁₀ alkyl polyglycoside, C₈₋₁₂ N,N-dimethyl-3-ammonio-1-propanesulfonate, and mixtures thereof. The antimicrobial composition(s) disclosed herein may comprise from 0.05% to 30% of surfactant, where the surfactant is selected from the group consisting of sodium octyl sulfate, sodium decyl sulfate, sodium octyl glyceryl ether sulfonate, sodium dodecyl methyl ester sulfonate, sodium dodecyl fatty acid sulfonate, octyl dimethyl amine oxide, octyl pyrrolidone, and mixtures thereof.

### Acidifying Agent

The compositions disclosed herein comprises an acidifying agent. The acidifying agent may adjust the pH of the composition to the following range: from 1.0 to 6.0, or from 1.0 to 5.5, or from 1.0 to 5.0, or from 2.5 to 5.0. The acidifying agent may help stabilize the pH of the composition by providing buffering capacity. The acidifying agent may also sequester transition metals, including iron, copper, manganese and the like. The acidifying agent may be chosen to further enhance the antimicrobial activity of the composition. The acidifying agent may be a US EPA/Health Canada registered active or a European notified antimicrobial substance.

The acidifying agent may comprise an organic acid, an inorganic acid, or a mixture thereof. The acidifying agent may be substantially free of trace transition metal impurities. Suitable inorganic acids include phosphoric acid, sulfuric acid, urea-sulfuric acid, hydrochloric acid, sulfamic acid, methyl sulfuric acid, hypochlorous acid, sodium bisulfate, and the like. Suitable organic acids include polymeric acids comprising at least 3 carboxylic acid groups, C₁-C₁₁ organic acids comprising at least one carboxylic acid group, and organic acids that do not comprise carboxylic acid functional groups (such as imidazole derivatives or phenolic or polyphenolic compounds). Non-limiting examples of polymeric acids include polymers of acrylic acid, methacrylic acid, maleic acid, or itaconic acid or copolymers of acrylic acid, methacrylic acid, maleic acid, itaconic acid, or mixtures thereof. Polymeric acids may be homopolymers or copolymers having a molecular weight of 500 g/mol or greater. The polymeric acid may have a molecular weight ranging from 500 g/mol to 1,000,000 g/mol, or from 500 g/mol to 100,000 g/mol, or from 1,000 g/mol to 20,000 g/mol. Copolymers may be random copolymers or block copolymers. In addition to monomer units comprising carboxylic acid groups, the copolymers may also include one or more other monomers, such as styrene, acrylic ester, acrylamide, olefin sulfonate, and olefin acetate.

Non-limiting examples of C₁-C₁₁ organic acids include formic acid, acetic acid, benzoic acid, malonic acid, citric acid, maleic acid, fumaric acid, succinic acid, lactic acid, malic acid, tartaric acid, gluconic acid, glutaric acid, adipic acid, 2-ethyl-1-hexanoic acid, heptanoic acid, octanoic acid, nonanoic acid, undecylenic acid, butane tetracarboxylic acid, and the like. The organic acid may be derived from a renewable, plant-based feedstock and produced using natural processes, such as fermentation; examples include bio-based acetic acid, bio-based citric acid, bio-based lactic acid and bio-based succinic acid, and the like. The organic acid may have food-use pedigree or be Generally Regarded As Safe (GRAS) or a food additive by the US Food & Drug Administration.

The composition(s) disclosed herein may comprise acidifying agent, where the acidifying agent is selected from the group consisting of formic acid, acetic acid, benzoic acid, malonic acid, citric acid, maleic acid, fumaric acid, hypochlorous acid, succinic acid, gluconic acid, glutaric acid, lactic acid, 2-ethyl-1-hexanoic acid, octanoic acid, nonanoic acid, peracetic acid, peroctanoic acid, undecylenic acid, and mixtures thereof, or the acidifying agent is selected from the group consisting of benzoic acid, citric acid, lactic acid succinic acid, maleic acid, succinic acid, octanoic acid, and mixtures thereof.

The compositions may comprise from 0.01% to 40%, or from 0.03% to 25%, or from 0.05% to 10% acidifying agent. A concentrated composition may comprise from 0.5% to 1%, or from 1% to 3%, or from 3% to 5%, or from 5% to 10%, or from 10% to 20%, or from 20% to 40% of acidifying agent. An increased concentration of acidifying agent increases the composition's reserve buffering capacity, which reduces pH fluctuation upon dilution. Partial neutralization of the acidifying agent to a pH value just below its pKa (e.g., 0.1 to 0.5 pH units below the acidifying agent's pKa) may also help to reduce pH fluctuation upon dilution. A concentrate may therefore be formulated at an increased pH, which may lead to an improved overall safety profile without compromising in-use (diluted) antimicrobial performance. A ready-to-use composition may comprise from 0.01% to 0.05%, or from 0.05% to 0.10%, or from 0.10% to 0.15%, or from 0.15% to 0.25%, or from 0.25% to 0.50% of acidifying agent. Ready-to-use compositions may comprise greater amounts of acidifying agent, e.g., greater than 0.50%, for example, to treat surfaces contaminated with mycobacteria, spore forming organisms, or biofilms.

Generally, an increased pH may improve the overall safety of the composition, enhance the compatibility of the composition with a larger variety of optional adjuncts, and increase the scope of applications for which the composition may be used. For compositions having pH 3.5 or greater, the acidifying agent may be selected from acidifying agents having pKa values greater than 4.0; non-limiting examples of such acidifying agents include acetic acid (pKa = 4.8), succinic acid (pKa 4.2), benzoic acid (pKa = 4.2), trans-cinnmaic acid (pKa = 4.4), p-coumaric acid (4-hydroxy cibnnamic acid, pKa = 4.6), octanoic acid (pKa 4.9), undecylenic acid (pKa 5.0), heptanoic acid (pKa = 5.1), nonanoic acid (pKa = 5.2), imidazole (pKa = 7.0), hypochlorous acid (pKa = 7.0) and mixtures thereof. Diprotic acid salts, such as the monosodium salt of maleic acid (pKa2 = 6.1), and triprotic acid salts, such as the mono- and dibasic salts of citric acid (pKa2 = 4.5, pKa3 = 6.4), may also be used to adjust the pH of the composition to pH 4.0 and greater.

The weight ratio of surfactant to acidifying agent in the composition may be from 50:1 to 1:50, or from 10:1 to 1:10, or from 5:1 to 1:5, or from 3:1 to 1:3.

The acidifying agent may be chosen to potentiate or provide antimicrobial properties. The acidifying agent may be selected from the group consisting of benzoic acid, citric acid, succinic acid, glycolic acid, lactic acid, octanoic acid, hypochlorous acid, peroxyacetic acid, peroxyoctanoic acid, and mixtures thereof. Acids characterized by reduced water solubility, including succinic acid, benzoic acid, cinnamic acid and octanoic acid, may be especially beneficial.

### Amide

The compositions disclosed herein may comprise an amide of formula I,

R¹-CO-NR²R³ (I)

where R¹ is selected from the group consisting of linear or branched, substituted or unsubstituted C₆-C₁₂, or C₆-C₁₀ hydrocarbyl groups, each of R² and R³ is independently selected from H, OH, a halogen, or C₁-C₆ linear or branched, substituted or unsubstituted hydrocarbyl groups.

The compositions disclosed herein may comprise from 0.01%, or from -0.03%, or from -0.05% to 15%, or to 25%, or to 40% by weight of an amide of formula I.

A concentrated composition may comprise from 1% to 40%, or from 1% to 25%, or from 1% to 15%, or from 1% to 8%, or from 1% to 5%, or from 1% to 3% by weight of an amide of formula I. A concentrated antimicrobial composition may comprise from 3% to 40%, or from 3% to 15%, from 3% to 8%, from 3% to 5% by weight of an amide of formula I. A concentrated antimicrobial composition may comprise from 5% to 40%, or from 5% to 15%, or from 5% to 8% by weight of an amide of formula I. A concentrated composition may comprise from 8% to 40%, or from 8% to 15% by weight of an amide of formula I. A concentrated composition may comprise from 15% to 40% by weight of an amide of formula I. A ready-to-use composition may comprise from 0.01% to 0.50%, or from 0.01% to 0.20%, or from 0.01% to 0.10%, or from 0.01% to 0.05% by weight of an amide of formula I. A ready-to-use composition may comprise from 0.05% to 0.50%, or from 0.05% to 0.20%, or from 0.05% to 0.10% by weight of an amide of formula I. A ready-to-use composition may comprise from 0.10% to 0.50%, or from 0.10% to 0.20% by weight of an amide of formula I. A ready-to-use composition may comprise from 0.20% to 0.50% by weight of an amide of formula I. Ready-to-use compositions may comprise greater than 0.50% by weight of an amide of formula I, for example, to treat surfaces contaminated with mycobacteria, spore forming organisms, or biofilms. The weight ratio of surfactant to amide of formula I may be from 0.05:1 to 10:1, or from 0.1:1 to 5:1, or from 0.2:1 to 5:1, or from 0.25:1 to 5:1.

The composition(s) disclosed herein may comprise surfactant, which comprises from 6 to 12 carbon atoms, and an amide of formula I, where the weight ratio of the surfactant to the amide of formula I is from 0.25:1 to 5:1.

Amides of formula I include monounsaturated amides, saturated amides, and hydroxamic acids. Non-limiting examples of amides of formula I include *n*-octanamide, N-hexyl-N-methyl decanamide, N,N-diethanol octanamide, N,N-dibutyl hexanamide, octanohydroxamic acid, and N,N-diethanol dodecanamide. The composition(s) disclosed herein may comprise amide of formula I, wherein the amide of formula I is selected from the group consisting of N,N-dimethyl octanamide, N,N-dimethyl decanamide, N,N-dimethyl 9-decenamide, N.N-dimethyl 7-octenamide, octanohydroxamic acid, and mixtures thereof. It is noted that C₆₋₁₂ hydroxamic acids, such as octanohydroxamic, may also provide chelation. For example, octanohydroxamic acid is known to have transition metal chelation properties, especially with respect to iron cations. As such, octanohydroxamic acid may be used, as a chelator, in combination with another amide of formula I, to supplement the activity of other amide. Combinations of C₆₋₁₂ hydroxamic acid or C₆₋₁₀ hydroxamic acid and another amide of formula I may be beneficial in promoting enhanced antimicrobial activity. Commercially available amides of formula I include Genagen 4296^{®}, an N,N-dimethyl decanamide available from Clariant, Steposol^{®} MET 10U, a N,N-dimethyl 9-decenamide available from Stepan Company, Cola^{®}Mid AL, a lauric acid N,N-diethanol amide available from Colonial Chemical, and octanohydroxamic acid available from TCI America. Additionally, Steposol^{®} M-8-10 is a mixture comprising approximately 55-60% N,N-dimethyl octanamide and approximately 40-45% N,N-dimethyl decanamide, which is derived from coconut oil and available from the Stepan Company.

It is believed that the amides disclosed herein potentiate the activity of antimicrobial actives against a variety of microorganisms, including Gram-positive bacteria, Gram-negative bacteria, non-enveloped viruses, fungi, mycobacteria, and even spore-forming organisms, such as *Clostridium difficile* spores. These potentiating effects are surprising given that amides alone are not known to have strong antimicrobial activity. Without wishing to be bound by theory, it is believed that the lipophilic character of the amide contributes to these potentiating effects; the amide is believed to preferentially partition into the microorganism, versus remaining in its monomer form in the composition. This partitioning is believed to induce micelles in the composition to release more amide monomers, in order to re-establish thermodynamic equilibrium. The released amide monomers again preferentially partition into the microorganism and the whole series of events - where amide monomers are continuously created from micelles and are then used up against the target microorganism - may contribute to the rapid antimicrobial activity of the disclosed compositions. By quickly and continuously permeating though microorganism defenses, the amide compound may also potentiate the activity of antimicrobial actives that are present in the composition.

For example, a composition that comprises hydrogen peroxide, as an antimicrobial active, may exhibit enhanced Fenton chemistry, with iron or copper from the microorganism, and may generate increased concentrations of oxygen-based radicals, which may react with the amide to form peracids or other highly reactive oxygen species (particularly, but not necessarily, inside the microorganism). For a composition comprising ionic silver, as an antimicrobial active, the nitrogen atom of the amide may associate with ionic silver, via a Lewis acid-base interaction, and may help to transport the silver ion into a microorganism, where it may cause death via known mechanisms.

The amide(s) disclosed herein may hydrolyze, over time, to its corresponding fatty acid, due to the acidic pH of the composition, particularly at a pH ranging from 1 to 2.5, and/or at increased temperatures (e.g., above room temperature). The compositions disclosed herein may therefore comprise a mixture of amide and its corresponding fatty acid. The fatty acid formed via hydrolysis may also contribute to antimicrobial activity, especially for compositions comprising hydrogen peroxide. For compositions comprising the amide of formula I and hydrogen peroxide, peracids may be formed via the following reactions:

R¹-C(O)N(R²)(R³) + H₂O = R¹C(O)OH + NH(R²)(R³);

and

R-C(O)OH + H₂O₂ = R-C(O)OOH + H₂O.

As amide hydrolysis is catalyzed by acid, increasing the pH of the composition may reduce amide hydrolysis, thereby reducing the concentration of peracid.

The level of fatty acid formed by amide hydrolysis may optionally be adjusted by the addition of from 0.1% to 10% of a lower alcohol, a primary C₁-C₆ amine, a secondary C₁-C₆ amine, a C₁-C₆ alkanol amine, or a mixture thereof to the composition. Suitable lower alcohols include methanol, ethanol, propylene glycol, dipropylene glycol, diethyleneglycol, glycerol, diglycerol, polyglycerol, or C₁ to C₈ mono- or di-glycerol ethers. The compositions disclosed herein may further comprise an ester. The compositions disclosed herein may comprise a mixture of amide(s), fatty acid(s) (e.g., generated via hydrolysis of the amide), and ester(s). Esters typically have desirable odor profiles.

### Water

The compositions disclosed herein may comprise water. The water may be of any hardness. The water may be de-ionized water, reverse-osmosis-treated water, distilled water, or soft water (typically, soft water does not exceed 40 ppm hardness (as CaCO₃)). The water may be de-ionized and/or reverse osmosis treated and may comprise less than 1 ppm transition metal ion, or less than 100 ppb transition metal ion.

The compositions may comprise from 15% to 99.95%, or from 20% to 95%, or from 20% to 90%, or from 25% to 85% water by weight of the composition. The amount of water in a given composition depends on the degree to which the composition is concentrated. A super concentrate composition may comprise less than 50%, or from 15% to 40%, or from 20% to 35% of water by weight of the composition. Such super concentrates may provide improved economics on a per-use basis (e.g., following recommended dilution) for the user. Also, the water activity of a super concentrate may be sufficiently reduced, such that the composition does not freeze at temperatures as low as -3°C, or as low as -18°C. Super concentrates may also exhibit improved ambient-temperature (e.g., 20-23°C) phase stability. Compositions comprising increased water content may also freeze more readily and exhibit phase instability upon thawing (e.g., crystallization or precipitation of one or more components). Super concentrate compositions may comprise surfactant, for example, a surfactant comprising from 10 to 12 carbon atoms; the surfactant may improve the ambient-temperature phase stability of the super concentrate compositions, upon dilution with water.

Ready-to-use compositions generally comprise greater water than concentrated compositions, which are intended to be diluted at the point of use. A ready-to-use composition may comprise from 70% to 99.9%, or from 75% to 99.5% water, or from 80% to 99% water by weight of the composition.

The composition(s) disclosed herein may comprise from 0.01% to 60%, or from 0.01% to 40% of surfactant, from 0.01% to 40% of acidifying agent, from 0.01% to 40% of amide of formula I, and from 15% to 99.95% of said water.

### pH

The compositions disclosed herein may have pHs ranging from 1.0 to 6.0, or from 1.0 to 5.5, or from 1.0 to 5.0, or from 2.5 to 6.0, or from 2.5 to 5.5, or from 2.5 to 5.0, or from 4.0 to 5.5.

For a concentrated composition that comprises less than 70% water, the pH is measured after adding de-ionized water to the composition, until the total concentration of water in the composition is 70%. For compositions that comprise greater than or equal to 70% water, pH is measured on the composition as made (the composition is not diluted prior to measuring the pH).

The composition(s) disclosed herein may comprise surfactant; acidifying agent; amide of formula I:

R¹-CO-NR²R³ (I)

where R¹ is selected from the group consisting of linear or branched, substituted or unsubstituted C₆-C₁₂, or C₆-C₁₀ hydrocarbyl groups, each of R² and R³ is independently selected from H, OH, a halogen, or C₁-C₆ linear or branched, substituted or unsubstituted hydrocarbyl groups; and water; where said composition has a pH from 1.0 to 6.0.

### Antimicrobial Active

The compositions herein comprise from 0.01% to 30% of an antimicrobial active. Said antimicrobial active is an active oxygen source, wherein the active oxygen source is hydrogen peroxide, and the active oxygen source is present at a level of from 0.05% to 8% by weight of the composition.

The composition preferably comprises from 0.06%, more preferably from 0.07% to 8% hydrogen peroxide. The concentration of hydrogen peroxide in the composition depends on the desired concentration of the overall composition (e.g., concentrate versus ready-to-use) as well as on the antimicrobial benefits sought.

Compositions comprising hydrogen peroxide may comprise less than 5 ppm transition metal ion impurities, or less than 2 ppm transition metal ion impurities, or less than 0.5 ppm transition metal ion impurities. Compositions comprising hydrogen peroxide may comprise less than 5 ppm ferrous ion, less than 5 ppm ferric ion, or less than 5 ppm of a mixture thereof, or less than 1 ppm ferrous ion, less than 1 ppm ferric ion, or less than 1 ppm of a mixture thereof, or less than 0.1 ppm ferrous ion, less than 0.1 ppm ferric ion, or less than 0.1 ppm of a mixture thereof.

A ready-to-use composition may comprise a greater concentration of hydrogen peroxide, e.g., greater than 1%, for example, for challenging antimicrobial benefits, such as the treatment of surfaces contaminated with mycobacteria, spore forming organisms, or biofilms.

The concentration of hydrogen peroxide is from 0.01% to 8.0%. The weight ratio of hydrogen peroxide to acidifying agent is from 0.1:1 to 10:1, or from 0.2:1 to 5:1, or from 0.5:1 to 2:1. The composition(s) disclosed herein may comprise acidifying agent and an antimicrobial active, where the antimicrobial active comprises hydrogen peroxide, where the weight ratio of the acidifying agent to the hydrogen peroxide is from 0.2:1 to 5:1.

The composition(s) disclosed herein may comprise amide of formula I and antimicrobial active, where the antimicrobial active comprises hydrogen peroxide, and in the amide of formula I, R¹ is selected from the group consisting of linear or branched, substituted or unsubstituted C₆-C₁₀ hydrocarbyl groups, wherein the weight ratio of hydrogen peroxide to the amide of formula I is from 0.2:1 to 5:1.

The combination of acid and hydrogen peroxide may generate measurable concentrations of peracid, from the reaction of acid and hydrogen peroxide.

The compositions disclosed herein comprise hydrogen peroxide and may be substantially free of C₆₋₁₂ peracids. The compositions disclosed herein may comprise catalytic amounts of peracid; in other words, the compositions disclosed herein may comprise from 1 ppm to 50 ppm, or 1 ppm to 10 ppm peracid, e.g., C₆₋₁₂ peracid.

The compositions disclosed herein may comprise hydrogen peroxide and peracid, where the peracid is formed in-situ via the reaction of a carboxylic acid-containing acidifying agent and hydrogen peroxide. For example, when the composition comprises octanoic acid or nonanoic acid, as the acidifying agent, there may be peroxyoctanoic acid or peroxynonanoic acid, respectively, formed in-situ in the composition. The rate of formation of the peracid may depend on the pH of the composition (reduced pHs favor peracid formation and faster rates of formation). The compositions disclosed herein may comprise hydrogen peroxide and peracid, where the peracid is formed in-situ via the reaction of the fatty-acid-product of amide hydrolysis and hydrogen peroxide. Peracid species may have various benefits, including antimicrobial benefits.

The compositions may comprise C₆₋₁₂ fatty acid, or C₆₋₁₀ fatty acid, or C₈ fatty acid (octanoic acid). The weight ratio of the fatty acid to the amide of formula I may be from 0.01:1 to 1:1, or from 0.05:1 to 0.5:1. The weight ratio of fatty acid to the corresponding peracid may be from 5:1 to 1000:1, or from 10:1 to 500:1, or from 15:1 to 100:1. The compositions may comprise a combination of C₆₋₁₂ peracid and a short-chain peracid (e.g., peracetic acid and peroctanoic acid).

The composition(s) disclosed herein may comprise amide of formula I, antimicrobial active, where the antimicrobial active comprises hydrogen peroxide, and C₆₋₁₀ fatty acid, where the weight ratio of the C₆₋₁₀ fatty acid to the amide of formula I is from 0.05:1 to 0.5:1.

The composition may further comprise one or more esters of formic acid, acetic acid, benzoic acid, lactic acid, succinic acid, 3-hydroxybutyric acid and citric acid, such as isobutyl formate, butyl acetate, ethyl benzoate, ethyl lactate, butyl 3-hydroxybutyrate, or triethyl citrate. The compositions may further comprise peracid from the in-situ reaction of acidifying agent with hydrogen peroxide, or peracid formed by hydrolysis/perhydrolysis of amides and esters in the composition; alternatively, the compositions may be substantially free of peracid, especially peracid formed from amide precursors of formula I.

The concentration of in-situ generated C₆₋₁₂ peracid in the composition may be from 0 ppm, or from .5 ppm, or from 1 ppm, to 10 ppm, or to 15 ppm, or to 25 ppm, or to 50 ppm. Surprisingly, the disclosed combination of surfactant, acidifying agent, and hydrogen peroxide, either in the absence of in-situ generated C₆₋₁₂ peracid or in combination with a low concentration of in-situ generated C₆₋₁₂ peracid, provides bactericidal activity at short exposure times (e.g., 15 seconds to 2 minutes). This surprising effect is demonstrated in the examples (compositions #15 and #16). The composition(s) disclosed herein may comprise an antimicrobial active, where the antimicrobial active comprises hydrogen peroxide, and from 1 to 50 ppm of C₆₋₁₀ fatty peracid. The composition(s) disclosed herein may comprise an antimicrobial active, where the antimicrobial active comprises hydrogen peroxide, and from 1 to 25 ppm peroctanoic acid.

The weight ratio of the surfactant to the antimicrobial active may be from 0.01:1 to 300:1, or from 0.1:1 to 100:1, or from 0.2:1 to 50:1. For compositions comprising ionic silver, as the optional antimicrobial active, the weight ratio of surfactant to ionic silver may be from 1:1 to 300:1, or from 2:1 to 200:1, or from 4:1 to 150:1, or from 10:1 to 100:1. For compositions comprising hydrogen peroxide, the weight ratio of surfactant to hydrogen peroxide may be from 0.05:1 to 20:1, or from 0.1:1 to 10:1, or from 0.2:1 to 5:1. For compositions comprising a combination of hydrogen peroxide and ionic silver, the weight ratio of hydrogen peroxide to ionic silver may be from 5:1 to 300:1, or from 10:1 to 250:1 or from 20:1 to 200:1. Weight ratios between components of the disclosed compositions may depend on a number of factors, including desired benefits and the optional adjuncts present in the composition.

The composition(s) disclosed herein comprise surfactant and antimicrobial active, where the antimicrobial active comprises hydrogen peroxide and the surfactant may comprise from 6 to 12 carbon atoms, and where the weight ratio of the surfactant to the hydrogen peroxide is from 0.1:1 to 10:1.

The compositions disclosed herein comprise hydrogen peroxide and may comprise ionic silver, or mixtures ionic silver and hydrogen peroxide in combination with an unregistered (North America) or unnotified (Europe) acidifying agent. The compositions disclosed herein comprises hydrogen peroxide and may comprise ionic silver, or mixtures of ionic silver and hydrogen peroxide in combination with a registered (North America) or notified (Europe) acidifying agent. The compositions disclosed herein may comprise benzoic acid, citric acid, glycolic acid, hypochlorous acid, lactic acid, octanoic acid, peroxyacetic acid, hydrogen peroxide, ionic silver, or mixtures thereof (which are US EPA and Health Canada registered antimicrobial actives). Benzoic acid, citric acid, lactic acid, hydrogen peroxide, and certain ionic silver compounds, such as silver nitrate, are also approved for use for water treatment or on food contact surfaces in the USA. Additionally, citric acid, l-lactic acid, ethanol, isopropanol, sodium bisulfate and hydrogen peroxide are the only antimicrobial approved actives for the US EPA's Design for the Environment (DfE) pesticide pilot project. Lactic acid, citric acid, peroxyoctanoic acid, and hydrogen peroxide are also notified substances in the European Union. These certifications may provide important credentialing options for the compositions disclosed herein.

The composition(s) disclosed herein may be a ready-to-use composition(s) comprising from 0.05% to 0.50% of surfactant; from 0.05% to 0.50% of acidifying agent; from 0.10% to 0.50% of antimicrobial active; from 0.05% to 0.50% of amide of Formula I. The composition(s) disclosed herein may be a ready-to-use composition(s) comprising from 0.05% to 0.50% of surfactant, where the surfactant comprises from 6 to 12 carbon atoms, or from 6 to 10 carbon atoms; from 0.05% to 0.50% of acidifying agent; from 0.10% to 0.50% of antimicrobial active, where the antimicrobial active comprises hydrogen peroxide; from 0.05% to 0.50% of amide of Formula I, where the amide of formula I is selected from the group consisting of N,N-dimethyl octanamide, N,N-dimethyl decanamide, N,N-dimethyl 9-decenamide, N.N-dimethyl 7-octenamide, octanohydroxamic acid, and mixtures thereof. The composition(s) disclosed herein may be a ready-to-use composition(s) comprising from 0.05% to 0.50% of surfactant, where the surfactant comprises 6 to 12 carbon atoms, or from 6 to 10 carbon atoms; from 0.05% to 0.50% of acidifying agent; from 0.002% to 0.1% of antimicrobial active, where the antimicrobial active comprises ionic silver; from 0.05% to 0.50% of amide of formula I, where the amide of formula I is selected from the group consisting of N,N-dimethyl octanamide, N,N-dimethyl decanamide, N,N-dimethyl 9-decenamide, N.N-dimethyl 7-octenamide, octanohydroxamic acid, and mixtures thereof.

The composition(s) disclosed herein may be a concentrated composition(s) comprising from 0.5% to 25%, or from 1% to 10% of surfactant; from 1% to 20%, or from 1% to 10% of acidifying agent; from 1% to 30%, or from 1% to 8% of antimicrobial active; from 1% to 35%, or from 1% to 10% of amide of formula I; and from 20% to 95%, or from 20% to 90% water. The composition(s) disclosed herein may be a concentrated composition(s) comprising from 0.5% to 25%, or from 1% to 10% of surfactant, wherein the surfactant comprises 6 to 10 carbon atoms; from 1% to 20%, or from 1% to 10% of acidifying agent; from 1% to 30%, or from 1% to 8% of antimicrobial active, wherein the antimicrobial active comprises hydrogen peroxide; from 1% to 35%, or from 1% to 10% of amide of formula I, where the amide of formula I is selected from the group consisting of N,N-dimethyl octanamide, N,N-dimethyl decanamide, N,N-dimethyl 9-decenamide, N.N-dimethyl 7-octenamide, octanohydroxamic acid, and mixtures thereof; and from 20% to 95%, or from 20% to 90% water.

The composition(s) disclosed herein may be a concentrated composition comprising from 0.5% to 25%, or from 1% to 10% of surfactant; from 1% to 20%, or from 1% to 10% of acidifying agent; from 0.005% to 0.5%, or from 0.005% to 0.05% of antimicrobial active; from 1% to 35%, or from 1% to 10% of amide of formula I; and from 20% to 95%, or from 20% to 90% water.

The composition(s) disclosed herein may be a concentrated composition comprising from 0.5% to 25%, or from 1% to 10% of surfactant, where the surfactant comprises from 6 to 10 carbon atoms; from 1% to 20%, or from 1% to 10% of acidifying agent; from 0.005% to 0.5%, or from 0.005% to 0.05% of antimicrobial active, where the antimicrobial active comprises ionic silver; from 1% to 35%, or from 1% to 10% of amide of formula I; and from 20% to 95%, or from 20% to 90% water.

### Adjuncts

The compositions disclosed herein may also contain one or more adjuncts. Adjuncts may be employed to increase immediate and/or residual efficacy of the compositions, improve the wetting characteristics of the compositions upon application to a target substrate, operate as solvents for diluted compositions, and/or serve to modify the aesthetic characteristics of the composition. These adjuncts may also provide degreasing and solubilizing benefits, additional antimicrobial potentiation, thickening, soil agglomeration or soil release benefits, enhanced composition solubility, further catalysis of antimicrobial activity, residual or long-lasting (e.g., 24 hours) duration antimicrobial properties and/or enhanced surface safety benefits.

The composition(s) disclosed herein may comprise an adjunct selected from the group consisting of chelants, builders, buffers, abrasives, electrolytes, bleaching agents, fragrances, dyes, foaming control agents, corrosion inhibitors, essential oils, thickeners, pigments, gloss enhancers, enzymes, detergents, solvents, dispersants, polymers, silicones, hydrotropes, and mixtures thereof.

### Solvents

The composition(s) disclosed herein may comprise a solvent (in addition to the amide of formula I, which may also function as a solvent). Solvents are generally liquid at ambient temperature conditions. Solvents may be desirable adjuncts especially for ready-to-use compositions and concentrated compositions, which are diluted with water at a ratio of 1 part concentrate to less than 10 parts water or 1 part concentrate to less than 5 parts water. The compositions disclosed herein may comprise from 0.25% to 25%, or from 0.5% to 15%, or from 1% to 10% of solvent by weight of the composition. Solvents may be used to control suds, adjust composition viscosity, or provide additional antimicrobial potentiation. Solvents may also be used to improve cleaning or prevent components of the composition from crystallizing out. Non-limiting examples of solvents that may improve cleaning include glycol ethers, more specifically C₁-C₈ derivatives of mono-, di-, and triethylene glycol ethers and diethers, and the C₁-C₆ derivatives of mono-, di- and tripropylene glycol ethers and diethers. Non-limiting examples include propylene glycol propyl ether, dipropylene glycol butyl ether, diethylene glycol butyl ether, tripropylene glycol dimethyl ether, ethylene glycol *n*-hexyl ether, ethylene glycol *n*-octyl ether, and the like. "Butyl" includes normal butyl, isobutyl and tertiary butyl groups. The solvent may be chosen to be non-VOC (Volatile Organic Compound), as defined by the California Air Resources Board, or VOC, e.g., ethanol, isopropanol and propylene glycol. A VOC solvent may be present at a concentration of less than 0.5% by weight of the in-use composition.

The composition(s) disclosed herein may comprise a solvent selected from the group consisting of ethanol, isopropanol, C₁-C₈ monoethylene glycol ether, C₁-C₈ diethylene glycol ether, C₁-C₈ triethylene glycol ether, C₁-C₆ monopropylene glycol ether, C₁-C₆ dipropylene glycol ether, C₁-C₆ tripropylene glycol ether, C₁-C₆ esters of formic acid, C₁-C₆ esters of acetic acid, C₁-C₆ esters of benzoic acid, C₁-C₆ esters of lactic acid, C₁-C₆ esters of 3-hydroxybutyric acid, C₁-C₆ amines, C₁-C₆ alkanol amines, and mixtures thereof. Examples of commercially available ethylene glycol-based solvents include Hexyl Cellosolve^{™} (ethylene glycol n-hexyl ether, C6 monoethylene glycol) and Butyl Carbitol^{™} (diethylene glycol n-butyl ether, C4 diethylene glycol) sold by Dow Chemical Company. Examples of commercially available propylene glycol-based solvents include Dowanol DPnB^{™} (dipropylene glycol *n*-butyl ether, C4 dipropylene glycol) and Dowanol TPM^{™} (tripropylene glycol methyl ether, C1 tripropylene glycol), which are also available from the Dow Chemical Company.

The composition(s) disclosed herein may comprise from 1% to 10% of a solvent selected from the group consisting of glycerol, diethylene glycol monoethyl ether, butyl 3-hydroxybutyrate, and mixtures thereof. Incorporation of diethylene glycol monoethyl ether, a non-VOC compound, may help solubilize highly crystalline and substantially water-insoluble materials, such as benzoic acid (acidifying agent) and octanohydroxamic acid (amide of formula I). Incorporation of diethylene glycol monoethyl ether may also improve freeze-thaw stability of the composition, particularly for compositions comprising benzoic acid, and/or octanohydroxamic acid, which are substantially water-insoluble, highly crystalline materials that may precipitate out or crystallize out when a composition is cooled during the freeze process or warmed during the thaw process.

For composition(s) having a pH of 2.5 or greater, or a pH of 3.0 or greater, an ester-based solvent may improve cleaning. Non-limiting examples of ester-based solvents include C₁-C₆ esters of formic acid, C₁-C₆ esters of acetic acid, C₁-C₆ esters of lactic acid, C₁-C₆ esters of citric acid, C₁-C₆ esters of succinic acid, and C₁-C₆ esters of 3-hydroxybutyric acid. The composition may comprise butyl 3-hydroxybutyrate (Omnia^{™} solvent, available from Eastman), which may provide a boost in cleaning performance, especially for greasy soils. Butyl 3-hydroxybutyrate may also help solubilize highly crystalline and substantially water-insoluble materials and promotes the freeze-thaw stability of the composition (particularly a composition comprising benzoic acid and/or octanohydroxamic acid).

Ready-to-use compositions comprising benzoic acid, octanohydroxamic acid, or mixtures thereof, may comprise greater than 0.5%, or greater than 1%, by weight of the composition, of ester-based solvent, such as butyl 3-hydroxybutyrate. At such concentrations, butyl 3-hydroxybutyrate may mitigate or prevent crystallization. Concentrated compositions may comprise from 3% to 10% of ester-based solvent, such that upon dilution, the in-use level is at least 0.5%, or at least 1% by weight of the in-use composition.

### Essential Oils

Suitable essential oils or actives thereof include those essential oils which exhibit antimicrobial activity. By "actives of essential oils" it is meant any ingredient of essential oils that exhibits antimicrobial activity. Essential oils and actives thereof may also provide a desirable odor profile.

Suitable essential oils include, but are not limited to, those obtained from thyme, lemongrass, citrus, lemons, oranges, anise, clove, aniseed, cinnamon, geranium, roses, mint, lavender, citronella, eucalyptus, peppermint, camphor, sandalwood, cedar, or mixtures thereof.

Actives of essential oils include, but are not limited to, thymol (present, for example, in thyme), eugenol (present, for example, in cinnamon and clove), menthol (present, for example, in mint), geraniol (present, for example, in geranium and rose), verbenone (present, for example, in vervain), eucalyptol and pinocarvone (present in eucalyptus), cedrol (present, for example, in cedar), anethol (present, for example, in anise), carvacrol, hinokitiol, berberine, terpineol, limonene, or mixtures thereof. The compositions disclosed herein may comprise thymol. Thymol is commercially available, for example, from Sigma Aldrich.

### Chelants

The compositions disclosed herein may comprise one or more chelants or sequestrants. As used herein, the terms "chelant" and "sequestrant" are used interchangeably. Chelants include chemical compounds that sequester alkali earth metal divalent ions, transition metal divalent ions, and/or transition metal trivalent ions from solution. The metal ions to be sequestered may be present in the compositions disclosed herein (for example, incorporated via hard water used in a dilution) or may be embedded within the microorganism that the composition is intended to treat. Metal ions in the concentrated compositions disclosed herein may originate from impurities in the water or in the raw materials used to make the compositions. These metal ions may adversely affect performance or composition stability. The concentration of metal ions may be reduced using processes to purify water, including reverse osmosis and de-ionization. Examples of such metal ions include the divalent and trivalent ions of iron, nickel, manganese, and the like.

Metal ions associated with a microorganism may be important for the functioning and survival of microorganism. The metal ions may be extracellular or they may be intracellular; the metal ions may be present, for example, at the active site of metabolic or regulatory enzymes or as a cofactor that enables enzymatic activity. Examples of metal ions associated with a microorganism include iron, copper, zinc and magnesium ions, and the like.

Highly water-soluble chelants may be used to sequester metal ions present in the composition. Lipophilic chelants may be used to target the metal ions associated with a microorganism. The composition(s) disclosed herein may comprise a mixture of hydrophilic and lipophilic chelants.

The composition(s) disclosed herein may comprise up to 10%, by weight of the composition, or from 1% to 10% of chelant. The chelant may comprise one or more phosphorus atoms. Non-limiting examples of phosphorous-containing chelants include 1-hydroxyethylidene-1,1-diphosphonic acid (HEDP, also known as etidronic acid), diethylene triamine penta(methylene phosphonic acid), 2-phosphonobutane-1,2,4-tricarboxylic acid (PBTC), and the like. The chelant may be a nil-phosphorus chelant. Non-limiting examples of nil-phosphorus chelants include the sodium, potassium, and alkanolamine salts of nitrilotriacetic acid (NTA), methyl glycine diacetic acid (MGDA), glutamic N.N-diacetic acid (GLDA), ethylene diamine tetraacetic acid (EDTA), diethylene triamine pentaacetic acid (DTPA), iminodisuccinic acid (IDS), ethylenediamine N,N'-disuccinic acid (EDDS), 4,5-dihydroxy-1,3-benzene disulfonic acid (Tiron), 2-hydroxypyridine N-oxide (HPNO), octyl isothiazolinone (OIT), picolinic acid, dipicolinic acid, 1-hydroxy-4-methyl-6-(2,4,4-trimethylpentyl) pyridine-2-one (piroctone acid), and the like. 2-hydroxypyridine N-oxide (HPNO) may be employed for chelation as well as for reducing precipitation or crystallization of crystalline components, such as benzoic acid or octanohydroxamic acid.

The composition(s) disclosed herein may comprise from 1% to 10% of a chelant selected from the group consisting of diethylene triamine pentaacetic acid (DTPA), iminodisuccinic acid, ethylenediamine N,N'-disuccinic acid, 4,5-dihydroxy-1,3-benzene disulfonic acid, octyl isothiazolinone, picolinic acid, dipicolinic acid, 2-hydroxypyridine N-oxide, and mixtures thereof.

The weight ratio of chelant to amide of formula I (when the amide is other than hydroxamic acid) may be from 1:30 to 1:3, or from 1:20 to 1:5. Incorporation of one or more chelants into the compositions disclosed herein may provide additional potentiation benefits, further supplementing the activity of the amide of formula I, particularly at greater composition pHs (e.g., pH 3 to 6 or pH 4 to 6).

### Wipe or pad

The present invention also relates to an article of manufacture comprising said composition, wherein the composition is comprised in a spray dispenser, or in a wipe or pad. The composition can be comprised on a wipe or pad. Such wipes and pads can be suitable for treating hard surfaces, such as found in the household, and the like. Suitable wipes can be fibrous. Suitable fibrous wipes can comprise polymeric fibres, cellulose fibres, and combinations thereof. Suitable cellulose-based wipes include kitchen wipes, and the like. Suitable polymeric fibres include polyethylene, polyester, and the like. Polymeric fibres can be spun-bonded to form the wipe. Methods for preparing thermally bonded fibrous materials are described in U.S. application Ser. No. 08/479,096 (Richards et al.), filed Jul. 3, 1995 (see especially pages 16-20) and U.S. Pat. No. 5,549,589 (Horney et al.), issued Aug. 27, 1996 (see especially Columns 9 to 10). Suitable pads include foams and the like, such as HIPE-derived hydrophilic, polymeric foam. Such foams and methods for their preparation are described in U.S. Pat. No. 5,550,167 (DesMarais), issued Aug. 27, 1996; and commonly assigned U.S. patent application Ser. No. 08/370,695 (Stone et al.), filed Jan. 10, 1995.

### Methods of Use

The compositions disclosed herein may be used in a variety of applications and methods, including the treatment of hard surfaces and the treatment of soft inanimate surfaces. The compositions may be used in the home to clean, sanitize, disinfect or sterilize hard surfaces, such as counters, sinks, restrooms, toilets, bath tubs, shower stalls, kitchen appliances, floors, windows, walls, furniture, phones, toys, drains, pipes, . The compositions may also be used in commercial establishments, such as hotels, hospitals, care homes, eating establishments, fitness centers, schools, office buildings, department stores, and prisons, to clean, sanitize, disinfect, or sterilize equipment, tools, food and medical preparation areas (in addition to the surfaces mentioned above that are common to both homes and commercial establishments). The compositions disclosed herein may be used to treat indoor as well as outdoor surfaces and may also be used to sanitize, disinfect, or sterilize soft inanimate surfaces, such as carpets, area rugs, curtains, upholstery, and clothes, in both the home or in commercial settings.

The compositions may be used to treat bacteria, non-enveloped or enveloped viruses, fungi, spores, or allergens on surfaces or in the air. The compositions may also be used to purify contaminated water. The compositions may also be used in agricultural applications in the treatment of weeds, fruits, plants, and animals, including cattle and horses, as well as carcasses. The compositions may be used to disinfect or sanitize indoor or outdoor non-food, indirect food, or food contact agricultural premises, buildings, including animal housing, pens, feed troughs, greenhouses, storage containers and the like. The compositions may be used to sanitize or disinfect equipment used in non-food, indirect food, or food contact indoor and outdoor settings, including equipment used in green houses (with or without ornamental or food crops), feed handling, hatcheries, ice dispensing, processing livestock feeding, milk processing, milking, mushroom houses, poultry processing or handling, transport vehicles, and the like.

Ready-to-use compositions may be housed in any container that allows for dispensing. Such containers may be metered to dispense a desired quantity or may include devices, such as caps, that allow the user to determine the level of dosing. Examples of containers include bottles, aerosols, pumps, and the like. Ready-to-use compositions may also be embedded in wipes or foams, such as melamine-formaldehyde foams. Such wipes may comprise woven and/or nonwoven substrates, where the substrates may include synthetic fibers, non-synthetic fibers, or mixtures of synthetic and non-synthetic fibers. As such, the wipe may optionally comprise cellulosic or non-cellulosic fibers, and, for high chemical resistance, may be in the form of a microfiber. The wipe may be a stand-alone or singly-formed substrate or a laminate of two or more substrates. Concentrates may be housed in any container as well. Concentrates may be dosed using mechanical or electrical pumps.

The present disclosure relates to an article of manufacture comprising the composition(s) disclosed herein in a spray dispenser or in a nonwoven substrate. The spray bottle may be a 2-chamber bottle in which, for example, the antimicrobial active is present in a first chamber and is separated from other formulation components present in the second chamber so as to mitigate or preclude reactivity of the antimicrobial active prior to use. Upon spraying, the contents of the 2 chambers are mixed together and sprayed as a uniform solution. The present disclosure also relates to a method of reducing the population of microorganism on a surface comprising the steps of applying an effective amount of the composition(s) disclosed herein to the surface and wiping the surface. The present disclosure also relates to a method of reducing population of microorganism on a surface comprising the steps of applying an effective amount of the composition(s) disclosed herein to the surface, where the composition contacts the surface for 30 seconds to 2 minutes, and wiping the surface.

### Examples

The bactericidal and fungicidal activity of the compositions of the present disclosure is quantified by the Association of Official Analytical Chemists (AOAC) Germicidal Spray Test (GST) Official Method 961.02, Germicidal Spray Products as Disinfectants (Official Methods of Analysis of the AOAC, 2009 Edition). Briefly, the GST is a carrier based method used to evaluate disinfection efficacy of aerosol/pump-based spray products and volatile liquid products for registration with regulatory agencies such as the US EPA and Health Canada. In this method, a series of glass slides ("carriers") are inoculated with a representative test organism and dried for 30 minutes (> 4 log inoculation). The carriers contacting the dried organism film are then sequentially treated with the spray product until thoroughly wet and are exposed for a finite contact time. After exposure, the carriers are sequentially transferred to a liquid subculture medium specifically selected to neutralize the test substance antimicrobial active and to recover any surviving test organism. The carriers are incubated and visually examined for the presence or absence of growth. Results are recorded as: number of carriers showing growth ÷ number of carriers tested. For example, a test result with 4 carriers showing growth out of 60 carriers tested would be recorded as 4/60; it is understood that a lower number of carriers showing growth is suggestive of a stronger performance. As such, comparisons may be made to differentiate the cidal efficacy of different compositions at a given contact time. In examples below, the exposure (contact) time for each experiment or group of experiments is provided.

For purposes of illustrating the benefits provided by the compositions disclosed herein, a series of 60 carrier tests vs. Gram (+) *Staphylococcus aureus,* a series of 60 carriers test vs. Gram (-) *Pseudomonas aeruginosa,* and a series of 30 carriers tests vs. fungi *Trichophyton mentagrophytes* are run. The benefits associated with the amides of formula I are measured by comparing the performance of compositions with and without the amide, and, in some cases, comparing the performance of compositions comprising amide versus compositions comprising known additives.

The compositions below are made up by mixing the components together. The concentration of each component in a given composition corresponds to the weight of the component, provided on an active basis, as a percent of the weight of the composition. The order in which components are added may be important. For example, the antimicrobial active, when present, may be added last and may be added at ambient conditions near room temperature (e.g., 20-23°C) to avoid unintentional chemical reactivity. The components may be added in the following order: de-ionized water, then surfactant, then acid(s). The composition may be heated to 50°-60°C to accelerate dissolution of components, especially succinic acid. Once cooled back to room temperature, the amide and optional antimicrobial active may be added under constant agitation to ensure solution homogeneity. All compositions in the examples below are clear or translucent solutions as made. The compositions are housed in amber HDPE bottles to protect from the effects of light during storage. All antimicrobial testing is conducted within 2 months of product making or less, e.g., within 1 month of product making. All samples are stored at ambient conditions (20-23°C) prior to testing.

Just prior to testing, the compositions are diluted in either de-ionized water or in 400 ppm hardness AOAC synthetic water expressed as CaCO₃. The methodology is described in the Association of Official Analytical Chemists (AOAC), Official Method 960.09, Germicidal and Detergent Sanitizing Action of Disinfectants, Preparation of Synthetic Hard Water, in Official Methods of Analysis of the AOAC, 2005 Edition.

For the Germicidal Spray Test results, the following abbreviations are used:
SA = *Staphylococcus aureus* ATCC 6538
PA = *Pseudomonas aeruginosa* ATCC 15442
TM = *Trichophyton mentagrophytes* ATCC 9533

Dilutions are made on a volume basis. For example, a 1:30 dilution means that a ready-to-test solution is made by combining with 29 milliliters of water for each milliliter of the composition to be diluted, and a 1:50 dilution means that a ready-to-test solution is made by combining 49 milliliters of water for each milliliter of the composition to be diluted. DI H₂O denotes de-ionized water. Hard water here denotes 400 ppm AOAC synthetic water expressed as CaCO₃. Carrier results are reported as the number of carriers showing growth divided by the total number of carriers.

Abbreviations:
C8 AS: Sodium octyl sulfate, tradename Stepanol^{®} C-8 sulfate from the Stepan Company, supplied as a 33% active solution in water.
C10 AS: Sodium decyl sulfate, tradename Polystep^{®} B-25 from the Stepan Company, supplied as a 38% solution in water.
C12 AS: Sodium lauryl sulfate, tradename Stepanol^{®} WA-Extra, supplied as a 30% solution in water.
DMA: dimethyl amide
Succ.: succinic acid; a bio-based succinic acid from the Succinity Corporation supplied as a > 99.5% powder.
C6 DMA: N,N-dimethyl hexanamide available from Frinton Laboratories, Hainesport, NJ as 98% active liquid
C8 DMA: N,N-dimethyl octanamide available from Adilab, West Newton, MA as a 95% active liquid
C8-10 DMA: N,N-dimethyl octanamide and N,N-dimethyl decanamide in a 55:45 weight ratio, tradename Steposol^{®} M-8-10 from the Stepan Company supplied as a near 100% active raw material.
C10 DMA: N,N-dimethyl decanamide, tradename Steposol^{®} M-8-10 from the Stepan Company supplied as a near 100% active raw material.
C12 DMA: N,N-dimethyl dodecanamide (lauric acid, N-N-dimethyl amide) from Santa Cruz Biotechnology, supplied as a near 100% active raw material.
Unsat. C10 DMA: N,N-dimethyl-9-decenamide (monounsaturated amide), tradename Steposol^{®} MET-10U form the Stepan company, supplied as a ≥ 97.0% active raw material.
Benzyl alcohol: Supplied by Lanxess Corporation as a ≥ 95% active raw material Silver nitrate: Supplied by Sigma-Aldrich as a ≥ 99.0% active raw material
Hydrogen peroxide: Supplied by Sigma-Aldrich as a 30% solution in water or as a 35% active raw material, tradename Interox URM 35-D from Solvay Chemicals.
C8 glycerol ester: Capmul^{®} 708G, a mixture of C8 mono glycerol ester (~88%) and higher esters (~12%); level of mono-dicaprylate ester ≥ 99%.
Butyl 3-Hydroxybutyrate: Omnia^{™} solvent, supplied by Eastman Chemical as a ≥ 98% active raw material,
Benzoic acid: Supplied by Sigma-Aldrich (ACS Reagent) as a ≥ 99,5% active crystalline powder raw material,
HPNO: 2-HydroxyPyridine N-oxide supplied by Sigma-Aldrich as a ≥ 96% active raw material C8 hydroxamic: Octanohydroxamic acid supplied by Tokyo Chemical Industry Ltd. as a ≥ 99.0% active raw material
NaOH: Sodium hydroxide, supplied as a 50% solution in water by Univar

### Compositions #1-4

Compositions # 1-4 are made and tested to evaluate the effect of C8-10 N,N-dimethyl amide on the antimicrobial activity of acidic compositions comprising hydrogen peroxide. Germicidal spray test evaluations are performed in DI water and hard water conditions. Testing results are at 1-minute exposure time (SA = *Staphylococcus aureus* ATCC 6538, PA = *Pseudomonas aeruginosa* ATCC 15442, TM = *Trichophyton mentagrophytes* ATCC 9533).

| | Composition | | | |
|---|---|---|---|---|
| Ingredient | #1^{∗} (wt%) | #2 (wt%) | #3 (wt%) | #4^{∗} (wt%) |
| C8 AS | 6.0 | 6.0 | 6.0 | 6.0 |
| Succinic Acid | 5.0 | 5.0 | 5.0 | 5.0 |
| Hydrogen | 4.5 | 4.5 | 4.5 | 4.5 |
| Peroxide | | | | |
| C8-10 DMA | --- | 6.0 | --- | --- |
| C10 DMA | --- | --- | 6.0 | --- |
| Benzyl Alcohol | --- | --- | --- | 6.0 |
| De-ionized Water | to 100 | to 100 | to 100 | to 100 |
| pH | 2.51 | 2.69 | 2.68 | 2.50 |

| Composition # | SA 1:30 dilution by wt DI H₂O | SA 1:50 dilution by wt DI H₂O | PA 1:50 dilution by wt DI H₂O | TM 1:10 dilution by wt DI H₂O |
|---|---|---|---|---|
| #1^{∗} | --- | 60/60 | 0/60 | 30/30 |
| #2 | 1/60 | 1/60 | 0/60 | 0/30 |
| #3 | 2/60 | 2/60 | 0/60 | 0/30 |
| #4^{∗} | 24/60 | 60/60 | 0/60 | 0/30 |

| Composition # | SA 1:30 dilution Hard H₂O | SA 1:50 dilution Hard H₂O | PA 1:50 dilution Hard H₂O | TM 1:10 dilution Hard H₂O |
|---|---|---|---|---|
| #1^{∗} | 60/60 | --- | 0/60 | 30/30 |
| #2 | 0/60 | 0/60 | 0/60 | 0/30 |
| #3 | 0/60 | 6/60 | 0/60 | 0/30 |
| #4^{∗} | 60/60 | 60/60 | 5/60 | 9/30 |

| | | | | |
|---|---|---|---|---|
| ^{∗} comparative | | | | |

Composition #1, which lacks the amide, shows 60 out of 60 carrier failures vs. *Staphylococcus aureus* at a 1:30 dilution and 30 out of 30 carrier failures at a 1:10 dilution vs. *Trichophyton mentagrophytes* in both DI water and hard water conditions. By contrast, composition # 2 and composition #3 (of the current application) show no more than 6 out of 60 carrier failures at a 1:50 dilution and no carrier failures vs. *Trichophyton mentagrophytes* at a 1:10 dilution in either hard DI water or hard water conditions. This indicates that the amide provides potentiation to the cidal activity provided by hydrogen peroxide. Composition #4 which lacks the amide but includes a known additive, benzyl alcohol, shows significantly weaker results versus the results achieved by compositions #2 and #3.

### Compositions #5-8

Compositions # 5-8 are made and tested to evaluate the effect of C8-10 N,N-dimethyl amide as compared to benzyl alcohol on the antimicrobial activity of acidic compositions comprising hydrogen peroxide. Germicidal spray test evaluations are performed in DI water and hard water conditions on *Staphylococcus aureus.* Compositions #5-8 contain succinic acid and a second organic acid, either lactic acid or glycolic acid. Testing results are at a 1-minute exposure time (SA = *Staphylococcus aureus* ATCC 6538).

| | | | | |
|---|---|---|---|---|
| | Composition | | | |

| Ingredient | #5 (wt%) | #6 (wt%) | #7^{∗} (wt%) | #8^{∗} (wt%) |
|---|---|---|---|---|
| C8 AS | 6.0 | 6.0 | 6.0 | 6.0 |
| Succinic Acid | 5.0 | 5.0 | 5.0 | 5.0 |
| Hydrogen | 4.5 | 4.5 | 4.5 | 4.5 |
| Peroxide | | | | |
| C8-10 DMA | 6.0 | 6.0 | --- | --- |
| Benzyl Alcohol | --- | --- | 6.0 | 6.0 |
| Lactic Acid | 6.0 | --- | 6.0 | --- |
| Glycolic Acid | --- | 4.5 | --- | 4.5 |
| De-ionized Water | to 100 | to 100 | to 100 | to 100 |
| pH | 2.02 | 1.94 | 1.80 | 1.76 |

| Composition # | SA 1:30 dilution DI H₂O | SA 1:50 dilution DIH₂O | SA 1:30 dilution Hard H₂O | SA 1:50 dilution Hard H₂O |
|---|---|---|---|---|
| #5 | 1/60 | 1/60 | 0/60 | 0/60 |
| #6 | 2/60 | 1/60 | 0/60 | 0/60 |
| #7^{∗} | 1/60 | 60/60 | 60/60 | --- |
| #8^{∗} | 3/60 | 29/60 | 3/60 | 51/60 |

| | | | | |
|---|---|---|---|---|
| ^{∗} comparative | | | | |

Composition #7, which is composition #4 with the addition of lactic acid, and composition #8, which is composition #4 with the addition of glycolic acid, show improved GST results, as compared to composition #4, on *Staphylococus aureus* at 1:30 dilution in DI water. However, composition #7 and composition #8 demonstrate weaker GST results than composition #5 and composition #6 (of the current application) for the remaining test conditions.

### Compositions #9-12

Compositions #9-12 are made and tested to evaluate the effect of different chain-length alkyl sulfates and different chain-length amides on the antimicrobial activity of acidic compositions comprising hydrogen peroxide. Testing results are at a 1-minute exposure time (SA = *Staphylococcus aureus* ATCC 6538, PA = *Pseudomonas aeruginosa* ATCC 15442, TM = *Trichophyton mentagrophytes* ATCC 9533).

| | Composition | | | |
|---|---|---|---|---|
| Ingredient | #9 (wt%) | #10 (wt%) | #11 (wt%) | #12 (wt%) |
| C10 AS | 6.0% | 6.0% | --- | --- |
| C12 AS | --- | --- | 6.0% | 6.0% |
| Succinic Acid | 5.0% | 5.0% | 5.0% | 5.0% |
| Hydrogen Peroxide | 4.5% | 4.5% | 4.5% | 4.5% |
| C8-10 DMA | 6.0% | --- | 6.0% | --- |
| C12 DMA | --- | 6.0% | --- | 6.0% |
| De-ionized Water | to 100% | to 100% | to 100% | to 100% |
| pH | 2.42 | 2.55 | 2.52 | 2.71 |

| Composition # | SA 1:30 dilution DI H₂O | SA 1:50 dilution DI H₂O | PA 1:50 dilution DI H₂O | TM 1:20 DI H₂O |
|---|---|---|---|---|
| #9 | 1/60 | 1/60 | 0/60 | 0/30 |
| #10 | 3/60 | 1/60 | 0/60 | 30/30 |
| #11 | 0/60 | 6/60 | 0/60 | 1/30 |
| #12 | 1/60 | 0/60 | 0/60 | 30/30 |

Each of composition #9, composition #10, compositions #11, and composition #12 is effective on *Staphylococcus aureus* and *Pseudomonas aeruginosa* up to a 1:50 dilution. Composition #10 and composition #12, both of which contain C₁₂ amide, are not effective on *Trichophyton mentagrophytes.* By comparison, composition #9 and composition #11, both of which contain C₈₋₁₀ amide, are effective on *Trichophyton mentagrophytes.* Composition #10 and composition #12, both of which contain C₁₂ amide, demonstrate weaker GST results than composition #2, which contains C₈₋₁₀ amide, but still improved GST results as compared to composition #1, which lacks an amide.

### Compositions #13-16

Compositions #13-16 evaluate the effect of C₈₋₁₀ N,N-dimethyl amide and C₈ glycerol ester on the antimicrobial activity of acidic compositions comprising hydrogen peroxide, at pH 3.0 as compared to pH 4.0 and in combination with HPNO as compared to lacking HPNO. Testing results are at a 1-minute exposure time (SA = *Staphylococcus aureus* ATCC 6538, TM = *Trichophyton mentagrophytes* ATCC 9533).

| | | | | |
|---|---|---|---|---|
| Composition | | | | |

| Ingredient | #13 (wt%) | #14 (wt%) | #15 (wt%) | #16^{∗} (wt%) |
|---|---|---|---|---|
| C8 AS | 6.0% | 6.0% | 6.0% | 6.0% |
| Succinic Acid | 5.0% | 5.0% | 5.0% | 5.0% |
| Hydrogen Peroxide | 4.5% | 4.5% | 4.5% | 4.5% |
| C8-10 DMA | 6.0% | 6.0% | 6.0% | --- |
| C8 Glycerol Ester | --- | --- | --- | 6.0% |
| Sodium Hydroxide | 0.25% | 1.0% | 1.0% | 1.0% |
| HPNO | --- | 0.5% | --- | 0.5% |
| De-ionized Water | to 100% | to 100% | to 100% | to 100% |
| pH | 3.01 | 4.01 | 4.01 | 4.02 |

| Composition # | SA 1:50 dilution DI H₂O | SA 1:50 dilution Hard H₂O | TM 1:20 DI H₂O | TM 1:20 Hard H₂O |
|---|---|---|---|---|
| #13 | 1/60 | 0/60 | 0/30 | 0/30 |
| #14 | 0/60 | 1/60 | 26/30 | 7/30 |
| #15 | 1/60 | 0/60 | 0/30 | 1/30 |
| #16^{∗} | 60/60 | 15/60 | 30/30 | 17/30 |

Each of composition #13, composition #14, and composition #15 is effective on *Staphylococcus aureus* at a 1:50 dilution in DI water and in hard water. However, only composition #13 (pH 3.0) and #14 (pH 4.0 with HPNO) are effective on *Trichophyton mentagrophytes* in DI water and in hard water. Comparison of the results for composition #14 versus composition #15 illustrates the effect that HPNO chelant has at pH 4.0. If one compares the effect of composition #15 versus the effect of composition #16 shows the benefits of the amide of formula I versus glycerol ester, at pH 4.0; this is unexpected given that esters are considered to be more reactive than amides.

Composition #15 is also analyzed and does not show detectable levels of hydrolysis after 1 month storage at ambient temperature conditions. By contrast, analytical testing of composition #16 shows detectable levels of acid hydrolysis after 1 month storage at ambient conditions; composition #16 contains partially hydrolyzed fatty acid ester (about 10% fatty acid). Yet, composition #16 demonstrates weaker GST results than composition #15. These results indicate that, surprisingly, the presence or formation of C₆₋₁₂ peracid in the composition(s) may not improve antimicrobial performance or that C₆₋₁₂ peracid may be present in catalytic amounts, e.g., 1 to 10 ppm.

The compositions disclosed herein may be free of, substantially free of, or comprise low concentrations of C₆₋₁₂ peracid, yet still provide improved antimicrobial activity - similar to or better than the antimicrobial activity of known peracid-comprising compositions. Importantly, by avoiding the generation of high concentrations of peracid, one may formulate effectively at increased pHs (e.g., pH of 3.0 to 6.0, or 3.5 to 6.0, or 4 to 5.5).

### Compositions #17-20

Compositions #17-20 are made and tested to evaluate the effect of C₈₋₁₀ N,N-dimethyl amide on the antimicrobial activity of acidic compositions comprising silver nitrate. Testing results are at a 1-minute exposure time time (SA = *Staphylococcus aureus* ATCC 6538, PA = *Pseudomonas aeruginosa* ATCC 15442, TM = *Trichophyton mentagrophytes* ATCC 9533).

| | | Composition | | |
|---|---|---|---|---|
| Ingredient | #17^{∗} (wt%) | #18^{∗} (wt%) | #19^{∗} (wt%) | #20^{∗} (wt%) |
| C8 AS | 6.0 | 6.0 | 6.0 | 6.0 |
| Succinic Acid | 5.0 | 5.0 | 5.0 | 5.0 |
| Silver Nitrate | 0.05 | 0.05 | 0.05 | 0.05 |
| C8-10 DMA | --- | 6.0 | --- | --- |
| C10 DMA | --- | --- | 6.0 | --- |
| Unsat. C10 DMA | --- | --- | --- | 6.0 |
| De-ionized Water | to 100 | to 100 | to 100 | to 100 |
| pH | 2.58 | 2.71 | 2.65 | 2.65 |

| Composition # | SA 1:30 dilution DI H₂O | PA 1:50 dilution DI H₂O | TM 1:5 DI H₂O | TM 1:20 DI H₂O |
|---|---|---|---|---|
| #17^{∗} | 60/60 | 0/60 | --- | 30/30 |
| #18^{∗} | 2/60 | 1/60 | --- | 17/30 |
| #19^{∗} | 2/60 | 0/60 | 0/30 | 1/30 |
| #20^{∗} | 2/60 | 0/60 | 1/30 | 16/30 |

| | | | | |
|---|---|---|---|---|
| ^{∗} comparative | | | | |

Composition #17, which lacks a dialkyl substituted amide, shows 60 out of 60 carrier failures versus *Staphylococcus aureus* at a 1:30 dilution and 30 out of 30 carrier failures at a 1:10 dilution versus *Trichophyton mentagrophytes.* Compositions #18, #19 and #20, each containing an amide of formula I, show improved results, as compared to Composition #17, versus *Staphylococus aureus* and *Trichophyton mentagrophytes.*

### Compositions #21-24

Compositions #21-24 are made and tested to evaluate the effect of C₈₋₁₀ N,N-dimethyl amide on the antimicrobial activity of acidic compositions comprising thymol. Germicidal spray test evaluations are performed versus *Staphylococcus aureus* (ATCC 6538) at a 1:30 dilution in de-ionized water using a 1 minute exposure time.

| | | Composition | | |
|---|---|---|---|---|
| Ingredient | #21^{∗} (wt%) | #22^{∗} (wt%) | #23^{∗} (wt%) | #24^{∗} (wt%) |
| C8 AS | 6.0 | 6.0 | 6.0 | 6.0 |
| Succinic Acid | 5.0 | 5.0 | 5.0 | 5.0 |
| C8-10 DMA | --- | 6.0 | --- | 6.0 |
| Acetic Acid | --- | --- | 4.5 | 4.5 |
| Thymol | 0.20 | 0.20 | 0.25 | 0.25 |
| De-ionized Water | to 100 | to 100 | to 100 | to 100 |
| pH | 2.61 | 2.72 | 2.42 | 2.53 |
| | #21^{∗} | #22^{∗} | #23^{∗} | #24^{∗} |
| Carrier failures | 60/60 | 10/60 | 17/60 | 4/60 |

| | | | | |
|---|---|---|---|---|
| ^{∗} comparative | | | | |

The results show that compositions containing an amide of formula I in combination with thymol and in the absence of ionic silver or hydrogen peroxide (composition #22 and composition #24), show improved antimicrobial results, as compared to composition #21 and composition #23, which contain thymol and no amide.

**Compositions #25-26**

| Ingredient | #25^{∗} (wt%) | #26 (wt%) |
|---|---|---|
| C8 AS | 6.0 | 6.0 |
| Succinic Acid | 5.0 | 5.0 |
| Hydrogen Peroxide | 4.5 | 4.5 |
| C8 Glycerol Ester | 6.0 | 6.0 |
| C8 Hydroxamic | --- | 0.5 |
| NaOH | 1.0 | 1.0 |
| De-ionized Water | to 100 | to 100 |
| pH | 3.93 | 4.02 |

| Composition # | SA 1:50 dilution DI H₂O | SA 1:50 dilution Hard H₂O |
|---|---|---|
| #25 | 40/60 | 60/60 |
| #26 | 1/60 | 0/60 |

| | | |
|---|---|---|
| ^{∗} comparative | | |

Composition #25, which lacks the amide of formula I, is not effective in the Germicidal Spray Test versus *Staphylococcus aureus* (ATCC 6538) at a 1:50 dilution. By contrast, composition #26, which includes 0.5% octanohydroxamic acid, passes the Germicidal Spray Test at a 1:50 dilution in DI and hard water conditions.

**Composition # 2 and # 27 Virucidal testing**

| Ingredient | #2 (wt%) | #27 (wt%) |
|---|---|---|
| C8 AS | 6.0 | 6.0 |
| Succinic Acid | 5.0 | 5.0 |
| Hydrogen Peroxide | 4.5 | 4.5 |
| C8-10 DMA | 6.0 | 6.0 |
| Acetic acid | --- | 4.5 |
| Water | to 100 | to 100 |
| pH | 2.69 | 2.52 |

The effectiveness of compositions #2 and #27 is evaluated versus three non-enveloped viruses as follows:
Rhinovirus Type 14 (RV 14) - ATCC VR-284, Poliovirus Type 1 (PV1) - ATCC VR-1562 and Feline calicivirus (FCV) - ATCC VR-782. Composition performance is measured using the well known EPA Virucidal Hard-Surface Efficacy Test. Briefly, 8 glass petri dishes with marked areas of 4 square inches are inoculated with 0.4 ml of the challenge microorganism and dried for 30 minutes. Inoculated carriers are then sprayed until thoroughly wet, from a distance of 6 to 8 inches, with each of compositions #2 and #27, which are pre-diluted 1:20 in AOAC hard water (400 ppm CaCO₃). The carriers include a 5% serum organic load and the exposure time is 1 minute at 20 ±2°C.

| | Composition #2 | | | Composition #27 | | |
|---|---|---|---|---|---|---|
| | 1:20 dilution, Hard H₂O | | | 1:20 dilution, Hard H₂O | | |
| | Carrier Result | Log Reduction | Conclusion | Carrier Result | Log Reduction | Conclusion |
| RV 14 | 0/8 failures | ≥ 4.13 | Passed | 0/8 failures | ≥ 4.13 | Passed |
| PV1 | 0/8 failures | ≥ 3.63 | Passed | 0/8 failures | ≥ 3.63 | Passed |
| FCV | 0/8 failures | ≥ 3.13 | Passed | 0/8 failures | ≥ 3.13 | Passed |

Compositions #2 and #27, diluted 1:20 in AOAC hard water, pass the Virucidal Hard-Surface Efficacy Test versus Rhinovirus Type 14, Poliovirus Type 1 *and Feline calicivirus.*

### Composition # 2 and # 27 Tuberculocidal Testing

The effectiveness of compositions #2 and #27 is evaluated versus *Mycobacterium bovis* (BCG), using the well known AOAC Germicidal Spray Test Tuberculocidal methodology. *Mycobacterium bovis* (BCG) is obtained from Organon Teknica Corporation. A one inch square area of each carrier is inoculated with 0.01 ml of the challenge microorganism and dried for 30 minutes. The inoculum contains a 5% serum organic load. Compositions #2 and #25 are diluted 1:10 (1 part composition to 9 parts diluent) using 400 ppm AOAC hard water. Inoculated carriers are then sprayed until thoroughly wet. Following 2 minutes exposure, the carriers are then sequentially transferred to a liquid subculture medium specifically selected to neutralize the test substance antimicrobial active and to recover any surviving test organism. The carriers are then incubated for 90 days and visually (morphology) and analytically (stain test) examined for the presence or absence of growth. The results are expressed as: number of tubes showing growth/total number of tubes.

| Composition | Day | Log (Avg. | Carrier Results |
|---|---|---|---|
| | | CFU/Carrier) | |
| #2 | 90 | 4.58 | 0/10 |
| #25 | 90 | 4.58 | 0/10 |

### Composition #2 and #27 Sporicidal Testing

The effectiveness of compositions #2 and #27 is evaluated versus *Clostridium difficile* in spore form - ATCC 43598, using the well known Standard Quantitative Disk Carrier Test Method. The *Clostridium difficile* in spore form includes an organic soil load. A 10 µl film of bacterial spores is dried on the surface of a brushed stainless steel disk. The inoculated and dried disk is placed into a Quantitative Carrier Test (QCT) vial and 50 µl of composition #2 or composition #27 is then applied to cover the inoculated carrier. After a 10 minute exposure time, the carriers are neutralized and quantitatively assayed for survivors. Results are shown below:

| Composition | Log (Avg. | Carrier Results | Log Reduction |
|---|---|---|---|
| | CFU/Carrier) | | |
| #2 | 6.43 | 0/10 | > 99.9999% |
| #25 | 6.14 | 0/10 | > 99.9999% |

Results show that compositions #2 and #25 are sporicidal at a 10 minute exposure time in the presence of organic soil.

### Compositions #28-31

Compositions #28-31 are made and tested to evaluate the effect of various short chain alkyl N,N-dimethyl amides on the antimicrobial activity of acidic compositions comprising cationic short chain amine oxide. Test results are obtained using a 1 minute exposure time (SA = *Staphylococcus aureus* ATCC 6538, TM = *Trichophyton mentagrophytes* ATCC 9533).

| Ingredient | #28^{∗} (wt%) | #29 (wt%) | #30 (wt%) | #31 (wt%) |
|---|---|---|---|---|
| C8 Amine Oxide | 6.0 | 6.0 | 6.0 | 6.0 |
| Succinic Acid | 5.0 | 5.0 | 5.0 | 5.0 |
| C6 DMA | --- | 6.0 | --- | --- |
| C8 DMA | --- | --- | 6.0 | --- |
| C10 DMA | --- | --- | --- | 6.0 |
| Hydrogen Peroxide | 4.5 | 4.5 | 4.5 | 4.5 |
| De-ionized Water | to 100 | to 100 | to 100 | to 100 |
| pH | 2.54 | 2.51 | 2.50 | 2.54 |

| Composition # | SA 1:50 dilution DI H₂O | SA 1:50 dilution Hard H₂O | TM 1:20 dilution DI H₂O | TM 1:20 dilution Hard H₂O |
|---|---|---|---|---|
| #28^{∗} | 59/60 | 60/60 | 30/30 | 30/30 |
| #29 | 1/60 | 0/60 | 0/30 | 0/30 |
| #30 | 0/60 | 0/60 | 6/30 | 5/30 |
| #31 | 2/60 | 9/60 | 1/30 | 27/30 |

| | | | | |
|---|---|---|---|---|
| ^{∗} comparative | | | | |

Results show that compositions comprising short chain cationic amine oxide and hydrogen peroxide do not provide strong antimicrobial results in the absence of amide compound, as compared to identical compositions that do comprise an alkyl amide compound. Best results are achieved with C6 dimethyl amide, followed by C8 dimethyl amide and finally C10 dimethyl amide.

### Compositions #32-35

Compositions #32-35 are made and tested to evaluate the effect of the C6 and C8 chain length N,N-dimethyl amides on the antimicrobial activity of acidic compositions comprising anionic short chain alkyl sulfate at pH 2.5 and pH 4.0. Test results are obtained using a 1 minute exposure time (SA = *Staphylococcus aureus* ATCC 6538, TM = *Trichophyton mentagrophytes* ATCC 9533).

| Ingredient | #32 (wt%) | #33 (wt%) | #34 (wt%) | #35 (wt%) |
|---|---|---|---|---|
| C8 Alkyl Sulfate | 6.0 | 6.0 | 6.0 | 6.0 |
| Succinic Acid | 5.0 | 5.0 | 5.0 | 5.0 |
| C6 DMA | 6.0 | --- | 6.0 | --- |
| C8 DMA | --- | 6.0 | --- | 6.0 |
| Hydrogen Peroxide | 4.5 | 4.5 | 4.5 | 4.5 |
| De-ionized Water | to 100 | to 100 | to 100 | to 100 |
| pH | 2.55 | 2.52 | 3.95 | 3.97 |

| Composition # | SA 1:50 dilution DI H₂O | SA 1:50 dilution Hard H₂O | TM 1:20 dilution DI H₂O | TM 1:20 dilution Hard H₂O |
|---|---|---|---|---|
| #32 | 0/60 | 0/60 | 0/30 | 0/30 |
| #33 | 0/60 | 0/60 | 0/30 | 0/30 |
| #34 | 0/60 | 0/60 | 0/30 | 0/30 |
| #35 | 0/60 | 0/60 | 0/30 | 0/30 |

Results show that compositions comprising short chain alkyl sulfate, hydrogen peroxide and C6 N.N-dimethyl amide or C8 N,N-dimethyl amide are fully effective vs. *Staphylococcus aureus* and *Trichophyton mentagrophytes* at pH ~2.5 and pH ~4.0 at the dilutions tested.

### Compositions #36-38

Compositions #36-38 are made and tested to illustrate the ability to tune antimicrobial potentiation achieved by the alkyl N,N-dimethyl amides of the invention by either raising or lowering the concentration of antimicrobial active Test results are obtained using a 1 minute exposure time (SA = *Staphylococcus aureus* ATCC 6538, TM = *Trichophyton mentagrophytes* ATCC 9533).

| Ingredient | #36 (wt%) | #2 (wt%) | #37 (wt%) | #38 (wt%) |
|---|---|---|---|---|
| C8 Alkyl Sulfate | 6.0 | 6.0 | 6.0 | 6.0 |
| Succinic Acid | 5.0 | 5.0 | 5.0 | 5.0 |
| C8-10 DMA | 6.0 | 6.0 | 6.0 | 6.0 |
| Hydrogen Peroxide | 3.0 | 4.5 | 6.0 | 7.5 |
| De-ionized Water | to 100 | to 100 | to 100 | to 100 |
| pH | 2.52 | 2.69 | 2.43 | 2.41 |

| Composition # | SA 1:50 dilution DI H₂O | SA 1:50 dilution Hard H₂O | SA 1:100 dilution DI H₂O | SA 1:100 dilution Hard H₂O |
|---|---|---|---|---|
| #36 | 2/60 | 1/60 | --- | --- |
| #2 | 0/60 | 0/60 | --- | --- |
| #37 | 0/60 | 0/60 | 1/60 | 0/60 |
| #38 | 0/60 | 0/60 | 0/60 | 0/60 |

| Composition # | TM 1:20 dilution DI H₂O | TM 1:20 dilution Hard H₂O | TM 1:50 dilution DI H₂O | TM 1:50 Dilution Hard H₂O |
|---|---|---|---|---|
| #36 | 26/30 | 30/30 | --- | --- |
| #2 | 0/30 | 0/30 | --- | --- |
| #37 | 2/30 | 0/30 | 0/30 | 0/30 |
| #38 | 0/30 | 0/30 | 0/30 | 0/30 |

Results show that the biocidal effectiveness of compositions comprising short chain alkyl sulfate, and alkyl N.N-dimethyl amide can be extended to higher dilution by increasing the level of antimicrobial active. That is, the potentiation of the amide compounds is not reduced when the concentration of antimicrobial active is increased.

### Compositions#39-40

Compositions #39-40 are made and tested to illustrate the ability to incorporate solvents while still delivering complete cidal effectiveness. Additionally, composition #40 uses benzoic acid to further illustrate flexibility in the invention with respect to selection of organic acid. Test results are obtained using a 1 minute exposure time (SA = *Staphylococcus aureus* ATCC 6538, TM = *Trichophyton mentagrophytes* ATCC 9533).

| Ingredient | #39 (wt%) | #40 (wt%) |
|---|---|---|
| C8 Alkyl Sulfate | 6.0 | 6.0 |
| Succinic Acid | 5.0 | --- |
| Benzoic Acid | --- | 4.0 |
| Hydrogen Peroxide | 4.5 | 4.5 |
| C8-10 DMA | 6.0 | 6.0 |
| Butyl 3-Hydroxybutyrate | 5.0 | 5.0 |
| De-ionized Water | to 100 | to 100 |
| pH | 3.97 | 3.36 |

| Composition # | SA 1:50 dilution DI H₂O | SA 1:50 dilution Hard H₂O | TM 1:20 dilution DI H₂O | TM 1:20 dilution Hard H₂O |
|---|---|---|---|---|
| #39 | 0/60 | 2/60 | 0/30 | 0/30 |
| #40 | 0/60 | 0/60 | 0/30 | 0/30 |

Results show that the cidal effectiveness of compositions comprising short chain alkyl sulfate, and alkyl N.N-dimethyl amide can be achieved in the presence of solvent and with alternative organic acids to succinic acid.

### Critical Micelle Concentration Data

The Critical Micelle Concentrations of the compositions shown below are measured at pH 2.5 ± 0.2 and/or pH 4.0 ± 0.2. The measurements are made using a Beckman-Coulter workstation model Biomek FX. Measurements are conducted on the complete compositions below and the results are for the complete compositions tested. All of the compositions tested are compositions of the present disclosure. Additionally, all of the compositions have Critical Micelle Concentrations between 100 ppm and 2,500 ppm, or between 150 ppm and 1,500 ppm. The CMC values are not materially affected by the presence of hydrogen peroxide or by the presence of HPNO chelant.

| Composition | pH | Water | CMC (ppm) |
|---|---|---|---|
| 6% C8 AS/ 5% Succ/ 6% C8-10 DMA^{∗} | 2.5 | DI | 1142 |
| 6% C8AS/ 5% Succ/ 6% C8-10 DMA/ 6% H2O2 | 2.5 | DI | 1037 |
| 6% C8 AS/5% Succ/ 6% C10 DMA/ 6% H2O2 | 2.5 | DI | 718 |
| 6% C8 AS/5% Succ/ 6% C12 DMA/ 6% H2O2 | 2.5 | DI | 159 |
| 6% C10 AS/ 5% Succ/ 6% C8-10 DMA^{∗} | 2.5 | DI | 856 |
| 6% C10 AS/ 5% Succ/ 6% C8-10 DMA/ 6% H2O2 | 2.5 | DI | 797 |
| 6% C10 AS/ 5% Succ/ 6% C10 DMA/ 6% H2O2 | 2.5 | DI | 537 |
| 6% C10 AS/ 5% Succ/ 6% C12 DMA/ 6% H2O2 | 2.5 | DI | 186 |
| 6% C12 AS/ 5% Succ/ 6% C8-10 DMA^{∗} | 2.5 | DI | 434 |
| 6% C12AS/ 5% Succ/ 6% C8-10 DMA/ 6% H2O2 | 2.5 | DI | 446 |
| 6% C8AS/ 5% Succ/ 6% C8-10 DMA/ 6% H2O2 | 4.0 | DI | 966 |
| 6% C8AS/ 5% Succ/ 6% C8-10 DMA/ 6% H2O2/ .5% | 4.0 | DI | 948 |
| HPNO | 4.0 | DI | 682 |
| 6% C8AS/ 5% Succ/ 6% C10 DMA/ 6% H2O2 | 4.0 | DI | 659 |
| 6% C8AS/ 5% Succ/ 6% C10 DMA/ 6% H2O2/ .5% HPNO | 4.0 | DI | 145 |
| 6% C8 AS/5% Succ/ 6% C12 DMA/ 6% H2O2 | 4.0 | DI | 140 |
| 6% C8 AS/5% Succ/ 6% C12 DMA/ 6% H2O2/ .5% HPNO | | | |
| 6% C10AS/ 5% Succ/ 6% C10 DMA/ 6% H2O2 | 4.0 | DI | 495 |
| 6% C10AS/ 5% Succ/ 6% C8-10 DMA/ 6% H2O2/ .5% | 4.0 | DI | 518 |
| HPNO | | | |
| 6% C12 AS/5% Succ/6% C8-10 DMA/ 6% H2O2 | 4.0 | DI | 446 |
| 6% C8 Amine Oxide/ 5% Succ/ 6% C8-10 DMA^{∗} | 4.0 | DI | 1107 |
| 6% C8 Amine Oxide/ 5% Succ/ 6% C8-10 DMA/ 6% H2O2 | 4.0 | DI | 975 |
| 6% C8 Amine Oxide/ 5% Succ/ 6% C10 DMA/ 6% H2O2 | 4.0 | DI | 590 |
| 6% C8 Amine Oxide/ 5% Succ/ 6% C12 DMA/ 6% H2O2 | 4.0 | DI | 181 |

| | | | |
|---|---|---|---|
| ^{∗} comparative | | | |

The CMC data indicate that the greatest driver for CMC is the chain-length of the amide - compositions containing longer-chain-length amides show significantly reduced CMC values. Another important driver of CMC is the chain-length of the surfactant.

## Claims

1. A composition comprising a surfactant; an acidifying agent; an amide of formula I:
R¹-CO-NR²R³ (I)
wherein R¹ is selected from the group consisting of linear or branched, substituted or unsubstituted C₆-C₁₂ hydrocarbyl groups, each of R² and R³ is independently selected from H, OH, a halogen, or C₁-C₆ linear or branched, substituted or unsubstituted hydrocarbyl groups; and water; wherein said composition has a pH from 1.0 to 6.0 wherein said composition is an antimicrobial composition comprising from 0.01% to 30% of an antimicrobial active, wherein the antimicrobial active is an active oxygen source, wherein the active oxygen source is hydrogen peroxide, and the active oxygen source is present at a level of from 0.05% to 8% by weight of the composition.

2. The composition according to claim 1, wherein said pH is from 2.5 to 5.0.

3. The composition according to any preceding claim, wherein said surfactant comprises from 6 to 12 carbon atoms.

4. The composition according to any preceding claim, wherein said composition comprises from 0.01% to 60% by weight of said surfactant, from 0.01% to 40% by weight of said acidifying agent, from 0.01% to 40% by weight of said amide of formula I, and from 15% to 99.95% by weight of said water.

5. The composition according to any preceding claim, wherein said composition has a critical micelle concentration from 100 ppm to 2,500 ppm.

6. The composition according to any preceding claim, wherein said surfactant is selected from the group consisting of C₈ glyceryl ether sulfonate, C₂-C₈ linear alkyl benzene sulfonate, C₆-C₁₂ alkyl sulfate, C₈-C₁₂ methyl ester sulfonate, C₈-C₁₂ fatty acid sulfonate, C₆-C₁₂ alkylethoxy carboxylate, C₆-C₁₂ alkylethoxy sulfate, C₈₋₁₀ dimethyl amine oxide, C₈ pyrrolidone, C₈ dimethyl betaine, C₈₋₁₀ alkyl polyglycoside, C₈₋₁₂ N,N-dimethyl-3-ammonio-1-propanesulfonate, and mixtures thereof.

7. The composition according to any preceding claim, wherein said composition comprises from 0.03% to 25% of said acidifying agent.

8. The composition according to any preceding claim, wherein said acidifying agent is selected from the group consisting of formic acid, acetic acid, benzoic acid, malonic acid, citric acid, maleic acid, fumaric acid, hypochlorous acid, succinic acid, gluconic acid, glutaric acid, lactic acid, 2-ethyl-1-hexanoic acid, cinnamic acid, heptanoic acid, octanoic acid, nonanoic acid, peracetic acid, peroctanoic acid, undecylenic acid, and mixtures thereof.

9. The composition according to any preceding claim, wherein said composition comprises from 0.03% to 25% of said amide of formula I.

10. The composition according to any preceding claim, wherein said amide of formula I is selected from the group consisting of N,N-dimethyl octanamide, N,N-dimethyl decanamide, N,N-dimethyl 9-decenamide, N,N-dimethyl 7-octenamide, octanohydroxamic acid, and mixtures thereof.

11. The composition according to any preceding claim, wherein in said amide of formula I, R¹ is selected from the group consisting of linear or branched, substituted or unsubstituted C₆-C₁₀ hydrocarbyl groups, wherein the weight ratio of said hydrogen peroxide to said amide of formula I is from 0.2:1 to 5:1.

12. The composition according to any preceding claim, wherein said composition comprises from 1 to 50 ppm of C₆₋₁₀ fatty peracid.

13. The composition according to any preceding claim, wherein said composition comprises a solvent selected from the group consisting of ethanol, isopropanol, C₁-C₈ monoethylene glycol ether, C₁-C₈ diethylene glycol ether, C₁-C₈ triethylene glycol ether, C₁-C₆ monopropylene glycol ether, C₁-C₆ dipropylene glycol ether, C₁-C₆ tripropylene glycol ether, C₁-C₆ esters of formic acid, C₁-C₆ esters of acetic acid, C₁-C₆ esters of benzoic acid, C₁-C₆ esters of lactic acid, C₁-C₆ esters of 3-hydroxybutyric acid, C₁-C₆ amines, C₁-C₆ alkanol amines, and mixtures thereof.

14. An article of manufacture comprising the composition according to any preceding claim, wherein the composition is comprised in a spray dispenser, or in a wipe or pad.

15. A method of treating a surface comprising the step of applying an effective amount of the composition according to claim 1 to said surface and optionally wiping said surface, preferably wherein said composition contacts said surface for 30 seconds to 2 minutes, before optionally wiping said surface, wherein said surface is a hard surface or a soft inanimate surface; wherein said hard surface is selected from the group consisting of counters, sinks, restrooms, toilets, bath tubs, shower stalls, kitchen appliances, floors, windows, walls, furniture, phones, toys, drains, pipes.

## Patentansprüche

1. Zusammensetzung, umfassend ein Tensid; ein Säuerungsmittel; ein Amid der Formel I:
R¹-CO-NR²R³ (I)
wobei R¹ ausgewählt ist aus der Gruppe bestehend aus linearen oder verzweigten, substituierten oder unsubstituierten C₆-C₁₂-Hydrocarbylgruppen, jedes von R² und R³ unabhängig ausgewählt ist aus H, OH, einem Halogen oder linearen oder verzweigten, substituierten oder unsubstituierten C₁-C₆-Kohlenwasserstoffgruppen; und Wasser; wobei die Zusammensetzung einen pH-Wert von 1,0 bis 6,0 aufweist, wobei die Zusammensetzung eine antimikrobielle Zusammensetzung ist, die zu von 0,01 Gew.-% bis 30 Gew.-% einen antimikrobiellen Wirkstoff umfasst, wobei der antimikrobielle Wirkstoff eine aktive Sauerstoffquelle ist, wobei die aktive Sauerstoffquelle Wasserstoffperoxid ist und die aktive Sauerstoffquelle in Höhe von 0,05 Gew.-% bis 8 Gew.-% der Zusammensetzung vorliegt.

2. Zusammensetzung nach Anspruch 1, wobei der pH-Wert von 2,5 bis 5,0 beträgt.

3. Zusammensetzung nach einem der vorstehenden Ansprüche, wobei das Tensid von 6 bis 12 Kohlenstoffatome umfasst.

4. Zusammensetzung nach einem der vorstehenden Ansprüche, wobei die Zusammensetzung zu von 0,01 Gew.-% bis 60 Gew.-% das Tensid, zu von 0,01 Gew.-% bis 40 Gew.-% das Säuerungsmittel, zu von 0,01 Gew.-% bis 40 Gew.-% das Amid der Formel I und zu von 15 Gew.-% bis 99,95 Gew.-% das Wasser umfasst.

5. Zusammensetzung nach einem der vorstehenden Ansprüche, wobei die Zusammensetzung eine kritische Mizellenkonzentration von 100 ppm bis 2.500 ppm aufweist.

6. Zusammensetzung nach einem vorstehenden Ansprüche, wobei das Tensid ausgewählt ist aus der Gruppe bestehend aus C₈-Glycerylethersulfonat, linearem C₂-C₈-Alkylbenzolsulfonat, C₆-C₁₂-Alkylsulfat, C₈-C₁₂-Methylestersulfonat, C₈-C₁₂-Fettsäuresulfonat, C₆-C₁₂-Alkylethoxycarboxylat, C₆-C₁₂-Alkylethoxysulfat, C₈₋₁₀-Dimethylaminoxid, C₈-Pyrrolidon, C₈-Dimethylbetain, C₈₋₁₀-Alkylpolyglycosid, C₈₋₁₂-N,N-Dimethyl-3-ammonio-1-propansulfonat und Mischungen davon.

7. Zusammensetzung nach einem der vorstehenden Ansprüche, wobei die Zusammensetzung zu von 0,03 % bis 25 % das Säuerungsmittel umfasst.

8. Zusammensetzung nach einem der vorstehenden Ansprüche, wobei das Säuerungsmittel ausgewählt ist aus der Gruppe bestehend aus Ameisensäure, Essigsäure, Benzoesäure, Malonsäure, Citronensäure, Maleinsäure, Fumarsäure, hypochloriger Säure, Bernsteinsäure, Gluconsäure, Glutarsäure, Milchsäure, 2-Ethyl-1-hexansäure, Zimtsäure, Heptansäure, Octansäure, Nonansäure, Peressigsäure, Peroctansäure, Undecylensäure und Mischungen davon.

9. Zusammensetzung nach einem der vorstehenden Ansprüche, wobei die Zusammensetzung zu von 0,03 % bis 25 % das Amid der Formel I umfasst.

10. Zusammensetzung nach einem der vorstehenden Ansprüche, wobei das Amid der Formel I ausgewählt ist aus der Gruppe bestehend aus N,N-Dimethyl-octanamid, N,N-Dimethyldecanamid, N,N-Dimethyl-9-decenamid, N,N-Dimethyl-7-octenamid, Octanohydroxamsäure und Mischungen davon.

11. Zusammensetzung nach einem der vorstehenden Ansprüche, wobei in dem Amid der Formel I R¹ ausgewählt ist aus der Gruppe bestehend aus linearen oder verzweigten, substituierten oder unsubstituierten C₆-C₁₀-Kohlenwasserstoffgruppen, wobei das Gewichtsverhältnis des Wasserstoffperoxids zu dem Amid der Formel I von 0,2:1 bis 5:1 beträgt.

12. Zusammensetzung nach einem der vorstehenden Ansprüche, wobei die Zusammensetzung zu von 1 bis 50 ppm C₆₋₁₀-Fettpersäure umfasst.

13. Zusammensetzung nach einem der vorstehenden Ansprüche, wobei die Zusammensetzung ein Lösungsmittel umfasst, das ausgewählt ist aus der Gruppe bestehend aus Ethanol, Isopropanol, C₁-C₈-Monoethylenglycolether, C₁-C₈-Diethylenglycolether, C₁-C₈-Triethylenglycolether, C₁-C₆-Monopropylenglycolether, C₁-C₆-Dipropylenglycolether, C₁-C₆-Tripropylenglycolether, C₁-C₆-Estern von Ameisensäure, C₁-C₆-Estern von Essigsäure, C₁-C₆-Estern von Benzoesäure, C₁-C₆-Estern von Milchsäure, C₁-C₆-Estern von 3-Hydroxybuttersäure, C₁-C₆-Aminen, C₁-C₆-Alkanolaminen und Mischungen davon.

14. Herstellungsartikel, umfassend die Zusammensetzung nach einem der vorstehenden Ansprüche, wobei die Zusammensetzung in einem Sprühspender oder in einem Wischtuch oder Pad enthalten ist.

15. Verfahren zum Behandeln einer Oberfläche, umfassend den Schritt des Auftragens einer wirksamen Menge der Zusammensetzung nach Anspruch 1 auf die Oberfläche und wahlweise Wischen der Oberfläche, wobei sich die Zusammensetzung vorzugsweise 30 Sekunden bis 2 Minuten lang mit der Oberfläche in Kontakt befindet, bevor die Oberfläche wahlweise gewischt wird, wobei die Oberfläche eine harte Oberfläche oder eine weiche unbelebte Oberfläche ist; wobei die harte Oberfläche ausgewählt ist aus der Gruppe bestehend aus Theken, Spülbecken, WCs, Toiletten, Badewannen, Duschkabinen, Küchengeräten, Fußböden, Fenstern, Wänden, Möbeln, Telefonen, Spielzeugen, Abflüssen, Rohren.

## Revendications

1. Composition comprenant un agent tensioactif ; un agent acidifiant ; un amide de formule I :
R¹-CO-NR²R³ (I)
dans laquelle R¹ est choisi dans le groupe constitué de groupes hydrocarbyle en C₆ à C₁₂ linéaire ou ramifié, substitué ou non substitué, chacun de R² et R³ est indépendamment choisi parmi H, OH, un halogène, ou des groupes hydrocarbyle linéaire ou ramifié, substitué ou non substitué, en C₁ à C₆ ; et de l'eau ; dans laquelle ladite composition a un pH de 1,0 à 6,0 dans laquelle ladite composition est une composition antimicrobienne comprenant de 0,01 % à 30 % d'un agent actif antimicrobien, dans laquelle l'agent actif antimicrobien est une source d'oxygène actif, dans laquelle la source d'oxygène actif est du peroxyde d'hydrogène, et la source d'oxygène actif est présente à un taux allant de 0,05 % à 8 % en poids de la composition.

2. Composition selon la revendication 1, dans laquelle ledit pH va de 2,5 à 5,0.

3. Composition selon une quelconque revendication précédente, dans laquelle ledit agent tensioactif comprend de 6 à 12 atomes de carbone.

4. Composition selon une quelconque revendication précédente, dans laquelle ladite composition comprend de 0,01 % à 60 % en poids dudit agent tensioactif, de 0,01 % à 40 % en poids dudit agent acidifiant, de 0,01 % à 40 % en poids dudit amide de formule I, et de 15 % à 99,95 % en poids de ladite eau.

5. Composition selon une quelconque revendication précédente, dans laquelle ladite composition a une concentration micellaire critique de 100 ppm à 2500 ppm.

6. Composition selon une quelconque revendication précédente, dans laquelle ledit agent tensioactif est choisi dans le groupe constitué de sulfonate d'éther de glycéryle en C₈, sulfonate d'alkylbenzène linéaire en C₂ à C₈, sulfate d'alkyle en C₆ à C₁₂, sulfonate de méthylester en C₈ à C₁₂, sulfonate d'acide gras en C₈ à C₁₂, carboxylate d'alkyléthoxy en C₆ à C₁₂, sulfate d'alkyléthoxy en C₆ à C₁₂, oxyde de diméthylamine en C_{8 à 10}, pyrrolidone en C₈, dyméthylbétaïne en C₈, alkyl polyglycoside en C_{8 à 10}, N,N-diméthyl-3-ammonio-1-propanesulfonate en C_{8 à 12}, et des mélanges de ceux-ci.

7. Composition selon une quelconque revendication précédente, dans laquelle ladite composition comprend de 0,03 % à 25 % dudit agent acidifiant.

8. Composition selon une quelconque revendication précédente, dans laquelle ledit agent acidifiant est choisi dans le groupe constitué d'acide formique, acide acétique, acide benzoïque, acide malonique, acide citrique, acide maléique, acide fumarique, acide hypochloreux, acide succinique, acide gluconique, acide glutarique, acide lactique, acide 2-éthyl-1-hexanoïque, acide cinnamique, acide heptanoïque, acide octanoïque, acide nonanoïque, acide peracétique, acide peroctanoïque, acide undécylénique, et des mélanges de ceux-ci.

9. Composition selon une quelconque revendication précédente, dans laquelle ladite composition comprend de 0,03 % à 25 % dudit amide de formule I.

10. Composition selon une quelconque revendication précédente, dans laquelle ledit amide de formule I est choisi dans le groupe constitué de N,N-diméthyl octanamide, N,N-diméthyl décanamide, N,N-diméthyl 9-décénamide, N,N-diméthyl 7-octénamide, acide octanohydroxamique, et des mélanges de ceux-ci.

11. Composition selon une quelconque revendication précédente, dans laquelle dans ledit amide de formule I, R¹ est choisi dans le groupe constitué de groupes hydrocarbyle linéaire ou ramifié, substitué ou non substitué en C₆ à C₁₀, dans laquelle le rapport pondéral dudit peroxyde d'hydrogène audit amide de formule I va de 0,2:1 à 5:1.

12. Composition selon une quelconque revendication précédente, dans laquelle ladite composition comprend de 1 à 50 ppm de peracide gras en C_{6 à 10}.

13. Composition selon une quelconque revendication précédente, dans laquelle ladite composition comprend un solvant choisi dans le groupe constitué d'éthanol, isopropanol, éther de monoéthylène glycol en C₁ à C₈, éther de diéthylène glycol en C₁ à C₈, éther de triéthylène glycol en C₁ à C₈, éther de monopropylène glycol en C₁ à C₆, éther de dipropylène glycol en C₁ à C₆, éther de tripropylène glycol en C₁ à C₆, esters en C₁ à C₆ d'acide formique, esters en C₁ à C₆ d'acide acétique, esters en C₁ à C₆ d'acide benzoïque, esters en C₁ à C₆ d'acide lactique, esters en C₁ à C₆ d'acide 3-hydroxybutyrique, amines en C₁ à C₆, alcanolamines en C₁ à C₆, et des mélanges de ceux-ci.

14. Article manufacturé comprenant la composition selon une quelconque revendication précédente, dans lequel la composition est comprise dans un atomiseur, ou dans une lingette ou un tampon.

15. Procédé de traitement d'une surface comprenant l'étape d'application d'une quantité efficace de la composition selon la revendication 1 à ladite surface et facultativement d'essuyage de ladite surface, de préférence dans lequel ladite composition vient en contact avec ladite surface pendant 30 secondes à 2 minutes, avant essuyage facultatif de ladite surface, dans lequel ladite surface est une surface dure ou une surface inanimée molle ; dans lequel ladite surface dure est choisie dans le groupe constitué de comptoirs, éviers, sanitaires, toilettes, baignoires, cabines de douche, appareils ménagers, sols, fenêtres, murs, meubles, téléphones, jouets, canalisations, tuyaux.
